# EUROPEAN PATENT APPLICATION

(11) **EP 4 725 950 A1**
(43) Date of publication of application: **15.04.2026**
(21) Application number: 25206790.5
(22) Date of filing: 06.10.2025
(51) Int. Cl.: C07H 1/00, C07H 1/06, C07H 21/00

(54) **IMPROVED SOLID-PHASE PHOSPHORAMIDITE-SYNTHESIS OF LONG OLIGONUCLEOTIDE MOLECULES**

(30) Priority: 08.10.2024 EP 24205357
(71) Applicant: BioSpring Gesellschaft für Biotechnologie mbH, 60386 Frankfurt am Main (DE)
(72) Inventor: SCHÄFER, Florian, 60386 Frankfurt am Main (DE); AYGÜN, Hüseyin, 60386 Frankfurt am Main (DE)
(74) Representative: Kraus & Lederer PartGmbB

(57) **Abstract**

The present invention relates to an improved method of a solid-phase phosphoramidite-synthesis for oligonucleotides, preferably RNA molecules having a length of between 50 and 500 nucleotides, as well as to an improved method of purification of said oligonucleotide molecules.

## Description

### FIELD OF THE INVENTION

The present invention relates to an improved method for the synthesis of oligonucleotide molecules in particular of long oligonucleotide molecules. The present invention, moreover, relates to an improved purification method of oligonucleotide molecules, in particular of long oligonucleotide molecules. The present invention is directed to an improved solid-phase phosphoramidite-synthesis of oligonucleotide molecules offering, among others, enhanced efficiency, scalability, yield and purity of the synthesized oligonucleotides. The method of the invention is applicable to a wide variety of oligonucleotide molecules, in particular of long RNA molecules, such as, for example, single guide RNAs, prime editing guide RNAs, engineered prime editing guide RNAs, tRNAs, aptamers, multivalent siRNA passenger strands or short mRNA molecules. The method is compatible with automated synthesis platforms.

### BACKGROUND OF THE INVENTION

Various methods in molecular biology and nucleotide and nucleotide-based diagnostics to amplify, detect and analyze nucleic acids depend on chemically synthesized oligonucleotides which serve as primers and probes to amplify or to detect nucleic acid targets. Synthetic nucleic acids are also active ingredients in a variety of gene therapeutics or gene therapy. A particularly important and promising application is the use of RNA molecules as guide strands in genome editing techniques such as single guide RNA (sgRNA), prime editing guide RNA (pegRNA), engineered prime editing guide RNA (epegRNA). Further important uses are the use of oligonucleotides as aptamers or short mRNA molecules in processes of modifying gene expression.

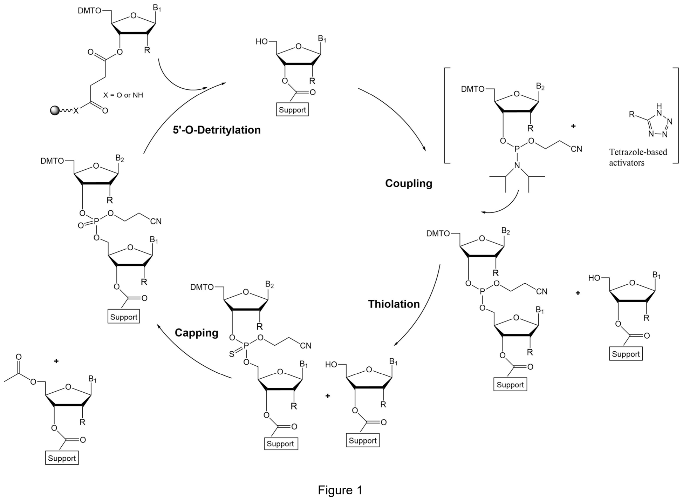

The phosphoramidite-synthesis method is the most widely used technique for synthesizing oligonucleotides. This method is highly efficient and allows for the rapid and automatic synthesis of RNA and DNA strands. Oligonucleotides are sequentially assembled from the 3'-end towards the 5'-end by deprotecting the 5'-end of the support-bound molecule, allowing the support-bound molecule to react with an incoming activated phosphoramidite building block. The resulting phosphite triester is then oxidized (e.g. sulphurized) to a phosphate (e.g. thiophosphate) triester and any unreacted hydroxyl groups are blocked by capping to prevent non-sequential coupling with the next incoming monomer. A general scheme of the synthesis cycle of the phosphoramidite synthesis is provided in Figure 1.

Methods of the phosphoramidite-synthesis are, for example, described in Usman et al. J. Am. Chem. Soc. 1987, 109, 7845 or Slim and Guide in Nucleic Acids Research 1991, 19, 1183-1188. These methods, however, are time consuming.

Moreover, the methods as described, for example, in US 2015/0218205 A1 or US 8,431,693 B2, provide the synthesis of oligonucleotides having up to 30 or maximum 40 nucleotide monomers.

There is, however, a need for synthesis methods of longer oligonucleotides in high yield and good quality.

It is, therefore, an object of the present invention to provide a production method and a purification method for oligonucleotides, in particular long oligonucleotides, which is not expensive, can be conducted quickly, is easily scalable and provides high production yields. That method should be particularly suitable for the synthesis of long oligonucleotide molecules having a length between 50 and 500 nucleotides. The process is to yield oligonucleotides, in particular long oligonucleotides having a high purity and low impurity levels. The process is to be compatible with automated synthesis platforms.

### SUMMARY OF THE INVENTION

As a solution to the above problem, the invention provides a method of solid phase phosphoramidite-synthesis of an oligonucleotide molecule comprising between 50 and 500 nucleotides, the method comprising at least the following steps:
a) providing a blueprint for the desired oligonucleotide sequence;
b) providing a solid support, preferably a base-loaded controlled pore glass support (CPG) preferably having a pore size from 1400 to 3000 A , functionalized with a linker moiety and providing a plurality of ribonucleotide monomer subunits as well as modified ribonucleotide monomer subunits, preferably 2'-modified and 4' modified ribonucleotide monomer subunits, locked nucleic acids and non-nucleosidic monomers, wherein each of the monomer subunits comprises a protected hydroxyl or specifically protected 5'- hydroxyl group;
c) treating the solid support for a predetermined time with an acidic deblocking solution to expose free 5'-hydroxyl groups;
d) washing the solid support with an appropriate solvent, preferably acetonitrile, to remove the deblocking solution, and the cleaved protecting group.
e) treating the solid support with a coupling mixture comprising an activating reagent and ribonucleotide monomer subunits, each comprising a phosphoramidite group and a blocked hydroxyl group to obtain phosphite triester linked ribonucleotide monomer subunits;
f) washing the solid support with an appropriate solvent, preferably acetonitrile, to remove the coupling mixture;
g) oxidizing or sulfurizing or thiolating the phosphite triester linked ribonucleotide monomer subunits to provide phosphate triester or thiophosphate triester linked ribonucleotide monomer subunits, whereby the oxidation or thiolation is carried out with an appropriate reagent;
h) washing the solid support with an appropriate solvent, preferably acetonitrile, to remove the oxidizing or thiolating solutions;
i) treating the phosphate triester or thiophosphate triester linked ribonucleotide monomer subunits with a mixture of one or more capping reagents to block any uncoupled support-bound 5' hydroxy byproduct, whereby the mixture of one or more capping reagents has less than 15, preferably less than 8, more preferably less than 5 equivalents of capping reagents based on the loading of the solid phase;
j) washing the solid support with an appropriate solvent, preferably acetonitrile, to remove the capping solutions;
k) repeating steps c) to j) until the desired amount of ribonucleotide units is coupled to the solid support to obtain the desired oligonucleotide molecule;
l) treating the solid support with a solution of an alkyl amine, a cyclic or a bicyclic amine in an appropriate solvent, preferably acetonitrile, to remove phosphorous protecting groups;
m) washing the solid support with an appropriate solvent, preferably acetonitrile, to remove the phosphorous deprotection reagent;
n) deprotecting and cleaving the oligoribonucleotide compound from the solid support to obtain the crude oligonucleotide molecule, and o) optionally, purifying the crude oligonucleotide molecule.

In an embodiment the above method of synthesis consists of the steps a) to o).

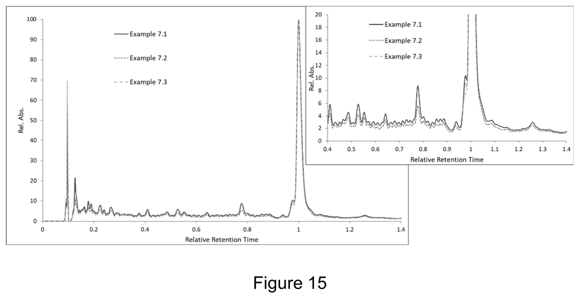

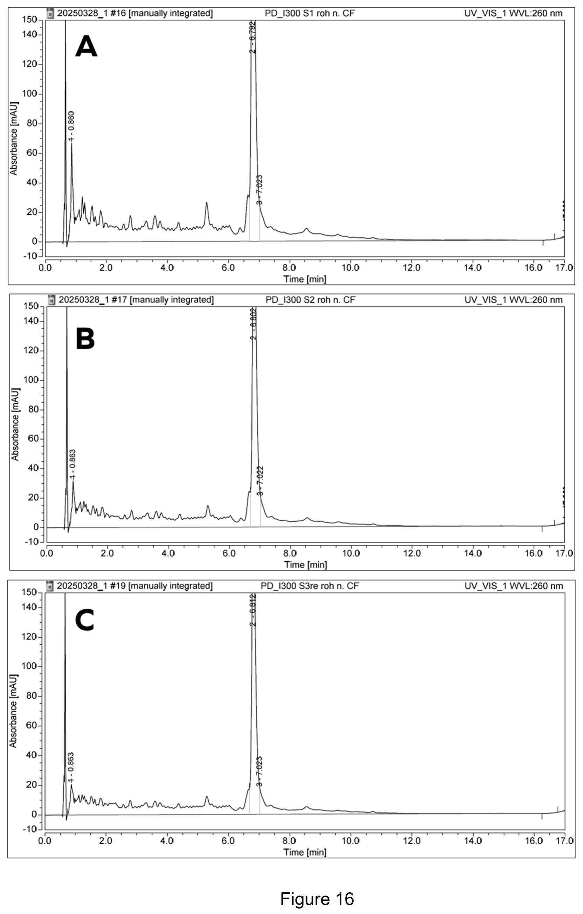

In a preferred embodiment for the first 50 cycles, more preferably for the first 10 cycles, even more preferably for the first 3 cycles, still more preferably for 3 to 25 cycles, the above steps e) and f) are carried out twice before step g) is conducted. In a preferred embodiment the deblocking solution in step c) comprises a predetermined amount of an organic acid, preferably dichloroacetic acid in an organic solvent, preferably toluene. The superior effect of a double coupling within the first 5 cycles and within the first 50 cycles is shown in Example 7 and Figures 15 and 16.

In more preferred embodiment the deblocking solution comprises dichloroacetic acid in an amount of between 1 and 10% wt.-%, based on the total weight of the deblocking solution, and the solid support is treated with the deblocking solution for a predetermined time of from 1 to 8 minutes, preferably less than 5 minutes.

In an even more preferred embodiment, the acidic deblocking solution comprises dichloroacetic acid in an amount between 2 and 6% wt% based on the total weight of the deblocking solution.

In a preferred embodiment the blocking group is DMT, MMT or TRT and deblocking solution is a detritylation solution. The activator of step e) can be a single activator or a mixture of several activators or multiple activators within the same synthesis. Preferably the activator is a single activating agent.

In a preferred embodiment the activating reagent of step e) is an acidic activator with a pKₐ < 7.0, and/or the concentration of nucleotide phosphoramidite is between 0.05 to 0.25 M. More preferably the concentration of the phosphoramidite is between 0.1 and 0.2 M. In a more preferred embodiment, the acidic activator is an acidic activator with a pKₐ between 5.0 and 5.5. More preferably the activator is selected from the group of imidazoles.

In a preferred embodiment the capping reagents of step i) are selected from N-Methylimidazole/Lutidine or picoline/Acetonitrile, tert.-Butylphenoxyacetic acid anhydride in acetonitrile, phenoxyacetic acid anhydride in acetonitrile, isobutyric anhydride in acetonitrile and acetic anhydride in acetonitrile.

In a preferred embodiment the deprotecting and cleaving step n) is carried out with a solution of ammonia or a basic alkylamine or a mixture thereof for 1 to 8 hours at a temperature ≤ 30°C, preferably ≤ 24°C. The basic amine is preferably methylamine. More preferably a 40% aqueous methylamine solution is used.

In a preferred embodiment the thiolation of step g) is conducted with 3-amino-1,2,4-dithiazole-5-thione (Xanthane hydride) or phenylacetyl disulfide and/or the oxidation is carried out with iodine in pyridine-water.

In a preferred embodiment on the top of the solid support a porous spacer plate is placed. Preferably said porous plate has a thickness of 0.2-10 cm, more preferably 1-3 cm. Preferably said porous spacer plate has a pore size equal to or above 20 µm, preferably equal to or above 80 µm. Most preferably the spacer plate has a thickness of 1 cm and a pore size of 80 µm and is made of polyethylene. Of course, a person skilled in the art will be in a position to determine the appropriate pore size depending on the respective conduct of the synthesis.

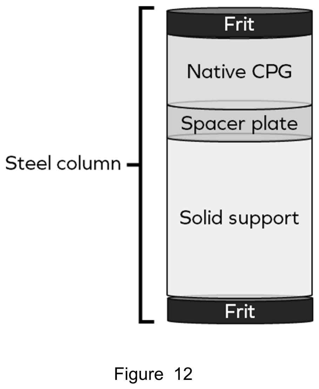

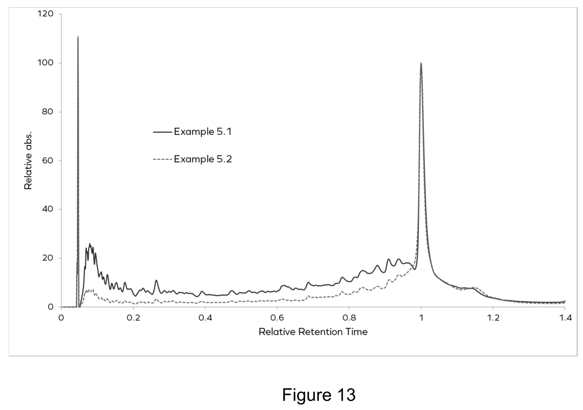

In a further preferred embodiment native controlled pore glass is filled on top of the porous spacer plate. It has been found that combining a porous spacer plate on top of the CPG support with native CPG on top of the porous spacer plate leads to a higher purity and increased yield of the obtained oligonucleotides (s. Example 5 and Figures 12 and 13).

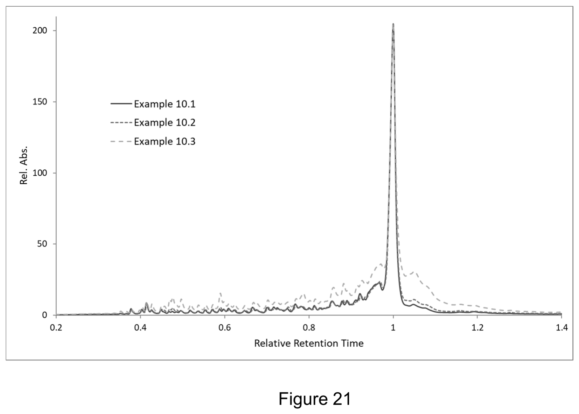

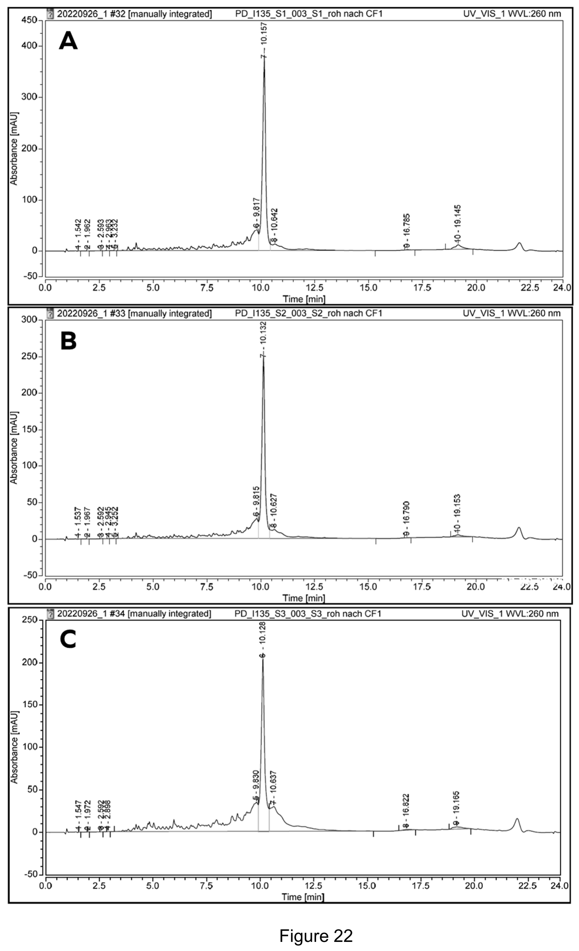

In a preferred embodiment the synthesis is conducted at a temperature between 10 and 28°C, preferably 10-25°C, preferably 14 - 28°C, more preferably between 18 and 24°C, still more preferably between 15 and 20°C (see Example 10 and Figures 21 and 22). The very most preferred temperature is between 18 and 24°C.In a preferred embodiment the purification of step o) is carried out according to the purification process below.

In a preferred embodiment of the above process and the purification process disclosed below the oligonucleotide molecule has a length between 60 and 250 nucleotides, more preferably between 70 and 170 nucleotides, even more preferably between 80 and 120 nucleotides.

In a preferred embodiment, an RNA molecule is prepared which has a length between 60 and 250 nucleotides, more preferably between 80 and 160 nucleotides.

The invention, moreover, provides a method for purifying an oligonucleotide molecule comprising between 50 and 500 nucleotides, synthesized by solid-phase phosphoramidite-synthesis comprising at least the steps
a) providing a crude oligoribonucleotide solution obtained by solid-phase phosphoramidite-synthesis;
b) optionally desalting the crude oligoribonucleotide solution;
c) purifying the desalted oligoribonucleotide solution by a chromatographic method or a combination of chromatographic methods, whereby a buffer having a pH of 3 to 8.5 is used and the chromatographic method is selected from anion exchange chromatography, hydrophobic interaction chromatography, ion exchange purification and/or ion pair - reversed phase purification or a combination thereof;
d) obtaining purified oligonucleotide molecule.
whereby the purification is carried out at a temperature between room temperature and 80°C.

In an embodiment of the invention the above method of purification consists of the steps a) to d).

In a preferred embodiment the phosphoramidite-synthesis is a method as disclosed above.

In a preferred embodiment the oligonucleotide molecule to be obtained is an oligonucleotide molecule obtained by the above process.

In a preferred embodiment the buffer having a pH of 3 to 8.5 is a phosphate buffer or an acetate buffer containing 5 to 30 %, preferably 10-20% of an organic solvent.

In a preferred embodiment purification of step c) is by anion exchange chromatography which is conducted at a temperature between 45 °C and 80°C, preferably between 45 and 70°C, more preferably between 50°C and 70°C (including the upper and lower limits).

In a preferred embodiment of the above method of synthesis and purification the oligonucleotide molecule is an RNA molecule.

In a preferred embodiment the RNA molecule is a single guide RNA (sgRNA), a prime editing guide RNA (pegRNA), an engineered prime editing guide RNA (epegRNA), a tRNA, an aptamer a multivalent siRNA passenger strand or a short mRNA.

### BRIEF DESCRIPTION OF THE DRAWINGS

**Figure 1****:** Basic scheme of the cycle of the phosphoramidite-synthesis.

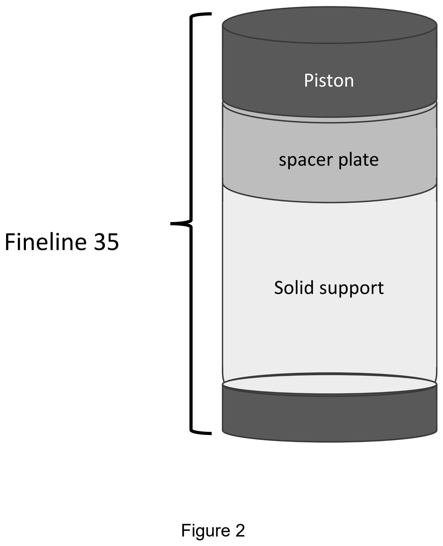

**Figure 2****:** Schematic drawing of Fineline 35 column with 1 cm porous spacer plate
**Figure 3****:** IP-HPLC analysis of 1.1 (upper chromatogram) and 1.2(lower chromatogram). FLP purity for 1.1: 24.75%, FLP purity for 1.2: 33.71%
**Figure 4****:** FLP purity for 2.1: 42.72 %, FLP purity for 2.2: 48.17 %, FLP purity for 2.3: 43.34 %, FLP purity for 2.4: 30.53 %.
**Figure 5****:** IP-HPLC analyses of 2.1 (A), 2.2 (B), 2.3 (C) and 2.4 (D). FLP purity for 2.1: 42.72 %, FLP purity for 2.2: 48.17 %, FLP purity for 2.3: 43.34 %, FLP purity for 2.4: 30.53 %.
**Figure 6****:** IP-HPLC analyses of 3.1 (: IP-HPLC analysis of 4.1, FLP purity 37.34 %, blue) and 3.2 (green). FLP purity for 3.1: 21.82 %, FLP purity for 3.2: 30.49 %.
**Figure 7****:** IP-HPLC analyses of 3.1 (A) and 3.2 (B). FLP purity for 3.1: 21.82 %, FLP purity for 3.2: 30.49 %.
**Figure 8****:** IP-HPLC analysis of 4.1, FLP purity 37.34 %.
**Figure 9****:** IP-HPLC analysis of 4.1, FLP purity 37.34 %.
**Figure 10****:** IP-HPLC analyses of 4.3.1 (blue), 4.3.2 (green) and 4.3.3 (orange). FLP purity for 4.3.1: 50.78 %, 4.3.2: 81.48 % and 4.3.3: 90.15 %.
**Figure 11****:** IP-HPLC analyses of purified synthesis 4.1 using ambient temperature 4.3.1 (A), elevated temperature (35-45°C) 4.3.2 (B) and high temperature (55-70°C) 4.3.3 (C). FLP purity for 3.1: 21.82 %, FLP purity for 4.3.1: 50.78 %, 4.3.2: 81.48 % and 4.3.3: 90.15 %.
**Figure 12****:** Schematic drawing of a 2.7 x 7 cm steel column with 1 cm porous spacer plate on top of the CPG and backfilled with native CPG.
**Figure 13****:** Normalized overlay of high resolving IP-HPLC analysis of 5.1 (upper chromatogram) and 5.2 (lower chromatogram). FLP purity for 5.1: 16.21 %, FLP purity for 5.2: 24.62 %.
**Figure 14****:** Normalized overlay of high resolving IP-HPLC analysis of 6.1 (upper chromatogram) and 6.2 (lower chromatogram). FLP purity for 6.1: 8.75 %, FLP purity for 6.2: 19.04 %.
**Figure 15****:** Normalized overlay of high resolving IP-HPLC analyses of 7.1 - 7.3. FLP purity for 7.1: 32.96 %, FLP purity for 7.2: 37.17 % and FLP purity for 7.3: 37.16 %.
**Figure 16****:** High resolving IP-HPLC analysis of 7.1 (A), 7.2 (B) and 7.3 (C). FLP purity for 7.1: 32.96 %, FLP purity for 7.2: 37.17 % and FLP purity for 7.3: 37.16 %.
**Figure 17****:** Normalized overlay of high resolving IP-HPLC analyses of 8.1 - 8.3. FLP purity for 8.1: 30.75 %, FLP purity for 8.2: 19.96 % and FLP purity for 8.3: 34.83 %.
**Figure 18****:** High resolving IP-HPLC analysis of 8.1 (A), 8.2 (B) and 8.3 (C). FLP purity for 8.1: 30.75 %, FLP purity for 8.2: 19.96 % and FLP purity for 8.3: 34.83 %.
**Figure 19****:** Normalized overlay of high resolving IP-HPLC analyses of 9.2.1 - 9.2.3. FLP purity for 9.2.1: 32.19 %, FLP purity for 9.2.2: 21.05 % and FLP purity for 9.2.3: 17.27 %.
**Figure 20****:** High resolving IP-HPLC analysis of 9.2.1 (A), 9.2.2 (B), 9.2.3 (C) and 9.2.4 (D). FLP purity for 9.2.1: 32.19 %, FLP purity for 9.2.2: 21.05 %, FLP purity for 9.2.3: 17.27 % and FLP purity for 9.2.4: 2.79 %.
**Figure 21****:** Normalized overlay of IP-HPLC analyses of 10.1 - 10.3. FLP purity for 10.1: 50.28 %, FLP purity for 10.2: 49.20 % and FLP purity for 10.3: 32.86 %.
**Figure 22****:** IP-HPLC analysis of 10.1 (A), 10.2 (B) and 10.3 (C). FLP purity for 10.1: 50.28 %, FLP purity for 10.2: 49.20 % and FLP purity for 10.3: 32.86 %.

### DETAILED DESCRIPTION OF THE INVENTION

Before the process of the invention will be described in detail, it is to be understood that this invention is not limited to specific process conditions or specific embodiments described herein, since such conditions and embodiments may, of course, vary.

It is also to be understood that the terminology used herein is not intended to be limiting, since the scope of the present invention will be defined only by the appended claims.

As used herein, the singular forms "a", "an", and "the" include both singular and plural referents unless the context clearly dictates otherwise. By way of example, "a compound" means one compound or more than one compound.

The terms "containing", "contains" and "contained of" as used herein are synonymous with "including", "includes" or " comprising", "comprises", and are inclusive or open-ended and do not exclude additional, non-recited members, elements or process steps. It will be appreciated that the terms "containing", "contains", "comprising", "comprises" and "comprised of" as used herein comprise the terms "consisting of", "consists" and "consists of".

Throughout this application, the term "about" is used to indicate that a value includes the standard deviation of error for the device or method being employed to determine the value.

As used herein, the term "average" refers to number average unless indicated otherwise.

As used herein, the terms "% by weight", "wt.- %", "weight percentage", or "percentage by weight" are used interchangeably. The same applies to the terms "% by volume", "vol.- %", "vol. percentage", or "percentage by volume", or "% by mol", "mol- %", "mol percentage", or "percentage by mol".

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different alternatives, embodiments and variants of the invention are defined in more detail. Each alternative and embodiment so defined may be combined with any other alternative and embodiment, and this for each variant unless clearly indicated to the contrary or clearly incompatible when the value range of a same parameter is disjoined. In particular, any feature indicated as being preferred or advantageous may be combined with any other feature or features indicated as being preferred or advantageous.

Furthermore, the particular features, structures or characteristics described in present description may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and from different embodiments, as would be understood by those in the art.

As used herein the term "monomer subunit" is meant to include all manner of monomer subunits that are amenable to oligomer synthesis. In general, a monomer subunit includes at least a sugar moiety having at least two reactive sites that can form linkages to further monomer subunits. Reactive sites on monomer subunits located on the termini of an oligomeric compound can be protected or unprotected (generally OH) or can form an attachment to a terminal group (conjugate or other group). Monomer subunits include, without limitation, nucleosides and modified nucleosides and can also be non-nucleosidic monomer subunits such as linker moieties. These non-nucleosidic monomer subunits can be contained in the oligonucleotide chain in variable amounts, at variable positions and/or in variable distribution over the oligonucleotide chain.

As used herein, the term "nucleoside" refers to a nucleobase-sugar combination. The two most common classes of such nucleobases are purines and pyrimidines. The term nucleoside includes β-D-ribonucleosides and β-D-2'-deoxyribonucleosides.

As used herein, the term "nucleotide" refers to a nucleoside further comprising a modified or unmodified phosphate internucleoside linking group or a non-phosphate internucleoside linking group. For nucleotides that include a pentofuranosyl sugar, the internucleoside linking group can be linked to either the 2',3' or 5' hydroxyl moiety of the sugar. The phosphate and or a non-phosphate internucleoside linking groups are routinely used to covalently link adjacent nucleosides to one another to form a linear polymeric compound.

As used herein the term "modified nucleoside" refers to a nucleoside comprising a modified heterocyclic base and or a sugar moiety other than ribose and 2'-deoxyribose. In certain embodiments, a modified nucleoside comprises a modified heterocyclic base moiety. In certain embodiments, a modified nucleoside comprises a sugar moiety other than ribose and 2'-deoxyribose. In certain embodiments, a modified nucleoside comprises a modified heterocyclic base moiety and a sugar moiety other than ribose and 2'-deoxyribose. The term "modified nucleoside" is intended to include all variants of modified nucleosides that can be incorporated into an oligomeric compound using standard oligomer synthesis protocols. Modified nucleosides include abasic nucleosides but in general a heterocyclic base moiety is included for hybridization to a complementary nucleic acid target.

The phosphoramidite-synthesis of oligonucleotide molecules involves the stepwise addition of nucleotide building blocks to a growing oligonucleotide chain. Each nucleotide is protected at reactive sites to prevent unwanted reactions during synthesis.

The present invention relates to a method of solid-phase phosphoramidite-synthesis of an oligonucleotide molecule comprising between 50 and 500 nucleotides, the method comprising at least the following steps:
a) providing a blueprint for the desired oligonucleotide sequence;
b) providing a solid support, preferably a base-loaded controlled pore glass support (CPG) preferably having a pore size from 1400 to 3000 A, functionalized with a linker moiety and providing a plurality of ribonucleotide monomer subunits as well as modified ribonucleotide monomer subunits, preferably 2'-modified and 4' modified ribonucleotide monomer subunits, locked nucleic acids and non-nucleosidic monomers, wherein each of the monomer subunits comprises a protected hydroxyl or specifically protected 5'- hydroxyl group;
c) treating the solid support for a predetermined time with an acidic deblocking solution to expose free 5'-hydroxyl groups;
d) washing the solid support with an appropriate solvent, preferably acetonitrile, to remove the deblocking solution and the cleaved protecting group.
e) treating the solid support with a coupling mixture comprising an activating reagent and ribonucleotide monomer subunits, each comprising a phosphoramidite group and a blocked hydroxyl group to obtain phosphite triester linked ribonucleotide monomer subunits;
f) washing the solid support with an appropriate solvent, preferably acetonitrile, to remove the coupling mixture;
g) oxidizing or thiolating the phosphite triester linked ribonucleotide monomer subunits to provide phosphate triester or thiophosphate triester linked ribonucleotide monomer subunits, whereby the oxidation or thiolation is carried out with an appropriate reagent;
h) washing the solid support with an appropriate solvent, preferably acetonitrile, to remove the oxidizing or thiolating solutions;
i) treating the phosphate triester or thiophosphate triester linked ribonucleotide monomer subunits with a mixture of one or more capping reagents to block any uncoupled support-bound 5' hydroxy byproduct, whereby the mixture of one or more capping reagents has less than 15, preferably less than 8, more preferably less than 5 equivalents of capping reagents based on the loading of the solid phase;
j) washing the solid support with an appropriate solvent, preferably acetonitrile, to remove the capping solutions;
k) repeating steps c) to j) until the desired amount of ribonucleotide units is coupled to the solid support to obtain the desired oligonucleotide molecule;
l) treating the solid support with a solution of an alkyl amine, a cyclic or a bicyclic amine in an appropriate solvent, preferably acetonitrile, to remove phosphorous protecting groups;
m) washing the solid support with an appropriate solvent, preferably acetonitrile, to remove the phosphorous deprotection reagent;
n) deprotecting and cleaving the oligoribonucleotide compound from the solid support to obtain the crude oligonucleotide molecule, and
o) optionally, purifying the crude oligonucleotide molecule.

The synthesis method of the present invention is basically applicable to the synthesis of any oligonucleotide molecule having any length. The method, however, is particularly suitable for the synthesis of long oligonucleotides. The method is particularly suitable for the synthesis of oligonucleotide molecules having a length between 50 and 500 nucleotides, preferably between 60 and 250 nucleotides, more preferably between 80 and 160 nucleotides. The oligonucleotide molecule may have any sequence and may be suitable for any purpose. Preferably an RNA molecule is synthesized. Preferred embodiments of RNA molecules are, for example, single guide RNAs (sgRNAs), prime editing guide RNAs (pegRNAs), engineered prime editing guide RNAs, tRNAs, multivalent siRNA passenger strands, aptamers or short mRNA molecules.

In a first step of the phosphoramidite-synthesis of the oligonucleotides of the invention, a blueprint for the desired sequence design is provided. This blueprint can also be referred to as the sequence design or sequence specification. It outlines the precise order of nucleotides that are needed to be assembled to form the target oligonucleotide molecule. It acts as a detailed plan guiding the synthesis process ensuring that each nucleotide is added in the correct sequence. This information is essential for the automatic synthesizer to accurately add nucleotides in the correct order ensuring that the final oligonucleotide product has the desired sequence and properties. This information can be taken from any source of information, i.e. an external source or an internal source, i.e. it can relate to already known sequences but may also relate to a completely newly designed sequence.

This first step of the solid-phase phosphoramidite-synthesis of an oligonucleotide molecule is a step that is per se known from the prior art and is generally applied for each and every method of solid-phase phosphoramidite-synthesis of oligonucleotide molecules.

The synthesis of the oligoribonucleotides is carried out on a solid support. A solid support for solid-phase phosphoramidite-synthesis is generally an insoluble material to which the first nucleotide of a growing oligonucleotide chain is covalently attached. This material provides a stable and inert platform that allows for the sequence addition of nucleotides in a controlled and efficient manner during the synthesis. The support can be a glass support, or a support made of a polymer. Usually, the solid support is composed of crosslinked polystyrene or controlled-pore-glass (CPG) both of which have functional groups that facilitate the attachment of the initial nucleotide. The use of the solid support enables easy separation of the growing oligonucleotide from the reactants and solvents simplifies purification steps and enhances the overall efficiency and yield of the synthesis.

Preferably, the solid support is a base loaded controlled-pore-glass support (CPG) functionalized with a linker moiety and preferably has a pore size from 1400 to 3000 A (Angstrom). The linker moiety is designed to be stable under the synthesis conditions yet cleavable under specific conditions to release the final oligonucleotide. Commonly used examples of linker moieties are succinyl linkers, long-chain alkyl amine linkers (LCAA), 4-4'-dimethoxy trityl linkers, trityl on / trityl off linkers, hydroquinone-O,O'-diacetic acid linkers (HQDA- or Q-linkers) etc. In a preferred embodiment native CPG may be filled on top of the porous spacer plate (s. Example 5, Figs. 12 and 13).

Then a plurality of ribonucleotide monomer subunits as well as -modified ribonucleotide monomer subunits, preferably 2' modified and 4' modified ribonucleotide monomer subunits, is provided. 2'-modified ribonucleotide monomer subunits are chemically altered nucleotides to improve stability, binding affinity or biological activity. Examples thereof are 2' O-methyl ribonucleotides, 2' fluoro ribonucleotides, 2' O-methoxyethyl ribonucleotides, locked nucleic acids, 2'amino ribonucleotides or 2' O-propargyl ribonucleotides. Basically, any type of 2'-modified ribonucleotide monomer subunit can be used according to the present invention. Preferred embodiments are 2'- fluoro, 2'- O-methyl modification, LNA, UNA, 2'-O- methoxy ethyl (MOE), linkers, modification as well as 2' deoxy-ribonucleotide monomers each of the monomer subunits comprises a protected hydroxyl or 5' hydroxyl group. The protection of the 5' hydroxyl group prevents unwanted reactions during the synthesis process. Commonly used examples of protecting groups for the 5' hydroxyl group are 4-4'-dimethoxy trityl (DMT), monomethoxy trityl (MMT), trityl (TRT),

However, also inversely protected subunits comprising a protected 3'-hydroxyl group can be used.

In the next step, the solid support is treated for a predetermined time with an acidic deblocking solution solution to expose free 5' hydroxyl groups for the next coupling step. Basically, any acid deblocking solution commonly used in the art can be used for the present synthesis method. The deblocking solution to be used depends on the nature of the blocking group used in the previous step.

In a preferred embodiment the deblocking solution comprises a predetermined amount of an organic acid, preferably dichloroacetic acid in an organic solvent, preferably toluene.

In more preferred embodiment the deblocking solution comprises dichloroacetic acid in an amount of between 1 and 10% wt.-%, based on the total weight of the deblocking solution.

In an even more preferred embodiment, the acidic deblocking solution comprises dichloroacetic acid in an amount between 2 and 6% wt% based on the total weight of the deblocking solution.

Examples of deblocking solutions are: 3% chloroacetic acid in toluene or dichloromethane, 3% dichloroacetic acid in dichloromethane, 2% dichloroacetic acid in dichloromethane, 3% trifluoroacetic acid in dichloromethane, 2% p-toluene sulfonic acid in acetonitrile, 2% trichloro acetic acid in toluene or 3% dichloroacetic acid in toluene. Said organic solvent is preferably toluene. The amount of the solvent and the flow rate of the solution depend among others from the conduct of the synthesis and the quality of the product to be obtained. These parameters can vary from step to step and can be adjusted by a person skilled in the art accordingly.

Preferably, the solid support is treated with the deblocking solution for a time from 1 to 8 minutes, preferably less than 5 minutes.

In a preferred embodiment the blocking group is DMT, MMT or TRT and deblocking solution is a detritylation solution.

In an even more preferred embodiment, the deblocking is carried out with a fixed column volume and a contact time of less than 5 min and a 3% DCA solution.

In an even more preferred embodiment, the deblocking is carried out with a fixed column volume and a contact time of less than 5 min and a 5% DCA solution.

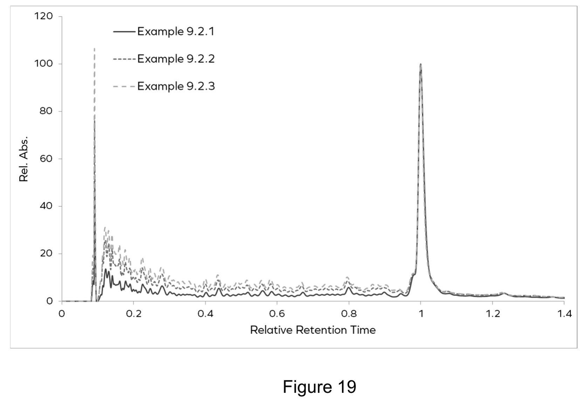

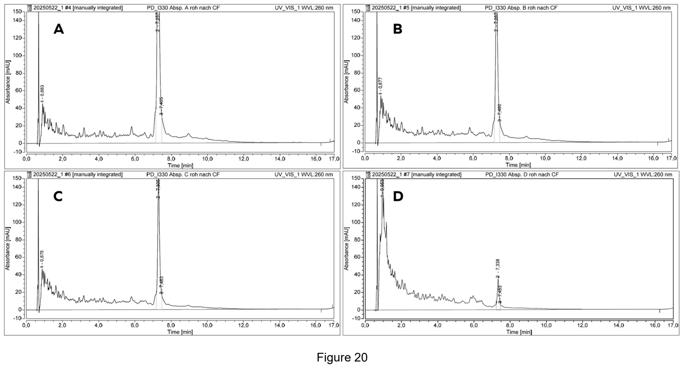

The effect of different cleavage and deprotection conditions is demonstrated in Example 9 and Figures 19 and 20.

In the next synthesis step (step e)), the solid support is treated with a coupling mixture, comprising an activating reagent and ribonucleotide monomer subunits, each comprising a phosphoramidite group and blocked hydroxyl group to obtain phosphite triester linked ribonucleotide monomer subunits. The activating agents are used to facilitate the coupling reaction between the phosphoramidite monomer and the growing solid phase bound chain.

Preferably, the activating reagent is an acidic activator with a pKₐ < 7.0, more preferably a pKₐ between 5.0 and 5.5, and is still more preferably chosen from the group of imidazoles. Examples of acidic activators with a pKₐ < 7.0 are tetrazole, 5(ethylthio)-1H-tetrazole, 4,5-dicyano imidazole or 2-benzylthio tetrazole and acid salts of pyridine or its derivatives (e.g. pyridinium trifluoroacetate). In a preferred embodiment the activating reagent is dicyanoimidazole.

Acidic activators with pKa values within the above range are known to a person skilled in the art and may be found in acknowledged textbooks such as p. Ex. In "Analysis of Oligonucleotides and their Related Substances" by George Okafo, David Elder, Mike Webb, ASIN : B00LBG519M Publisher : ILM Publications (5 Jun. 2013).

The activator can be a single component or a mixture of activators.The concentration of the nucleotide phosphoramidite in this step is preferably between 0.05 and 0.25 M, more preferably between 0.1 and 0.2 M, most preferably 0.2M. In a preferred embodiment for up to 50 cycles the coupling step e) is carried out twice before step g) is conducted.

The next synthesis steps comprise the capping and the oxidation reaction (steps g) and i)). Any unreacted sites on the solid support are capped to prevent unintended couplings and the phosphite triester linkage is oxidized / sulfurized / thiolated to form a stable phosphate linkage.

Basically, any commonly used oxidizing agents, sulfurizing agents or thiolating agents can be used.

Oxidizing agents are used to convert the phosphite triester linkage formed after the coupling step into a more stable phosphate triester linkage. Examples of oxidizing agents are iodine in water / pyridine / THF, tert-butylhydroperoxide or peracetic acid.

Sulfurizing agents are used to convert the phosphite triester linkage into a phosphorthiolate linkage which is more resistant to nucleases and often used in therapeutic oligonucleotides. Examples of sulfurizing agents are 3H-1,2 benzodithiol-3-one-1,1-dioxide (Beaucage reagent), phenylacetyl disulfide, or 3-Amino-1,2,4-dithiazole-5-thione (xanthane hydride).

Preferably, the oxidation is carried out with iodine in pyridine-water.

Moreover, alternative backbones to phosphate triester and phosphorthiolate linkage can be introduced at this stage, e.g guanidine-containing backbones by using for example 2-azido-1,3,dimethylimidazolinium hexaflurophosphate or other similar derivatives.

Corresponding methods are known in the art, p.Ex. from Skvortsova et al, Frontiers in Pharmacology, September 2019, Vol. 10, Artivle 1049, p. 1-9 ("A New Antisense Phosphoryl Guanidine Oligo-2'-Methylribonucleotide Penetrates into Intracellular Mycobacteria and Suppresses Target Gene Expression"), or from Kandasamy et al., Nucleic Acids Research, 2022, Vol. 50, No. 10, p. 5401-5423 ( Impact of guanidine-containing backbone linkages on stereopure antisense oligonucleotides in the CNS").

The phosphate triester or thiophosphate triester linked ribonucleotide monomer subunits are then treated with a mixture of one or more capping reagents (or a single reagent in case of a premixing of necessary chemicals). The capping reagent ensures that any unreacted 5'-OH groups are blocked so as to allow the next cycle of the reaction to proceed effectively. Basically, any capping reagents generally known in the art can be used. Preferably, the one or more capping reagents are selected from N-Methylimidazole/Lutidine or Picoline/Acetonitrile, tert.-Butylphenoxyacetic acid anhydride in acetonitrile, phenoxyacetic acid anhydride in acetonitrile, isobutyric anhydride in acetonitrile and acetic anhydride in acetonitrile. Preferred combinations are a solution of tert-Butylphenoxyacetic acid anhydride in acetonitrile combined with a solution of -N-Methylimidazole/Lutidine/Acetonitrile or a combination of acetic acid anhydride in acetonitrile combined with a solution of N-Methylimidazole/Lutidine/Acetonitrile. Preferably acetic acid anhydrides and its derivatives are used.

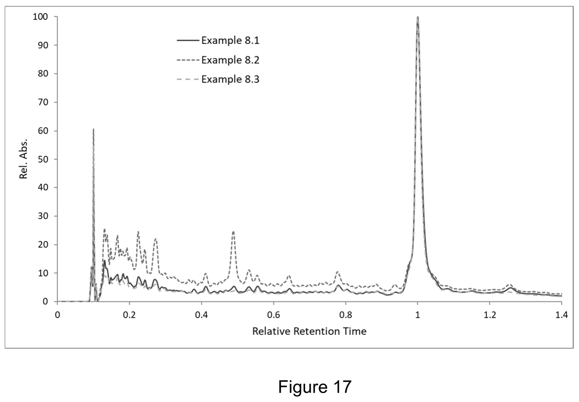

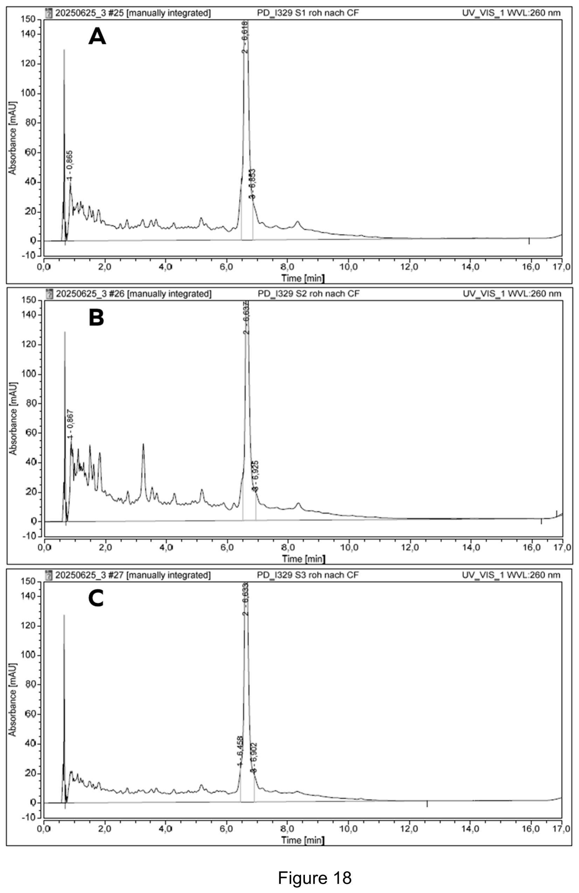

The mixture of the at least two capping reagents has less than 15, preferably less than 10, more preferably less than 8, even more preferably less than 5 equivalents of capping reagents based on the loading of the solid phase. Most preferably 1 to 9 or 2 to 9 equivalents are used. The effect of the use of different capping equivalents of tert-Butylphenoxyacetic acid anhydride capping reagent is shown in Example 8 and Figures 17 and 18.

Alternatively, the equivalent capping reagents can also be sequentially reduced throughout the synthesis. For example, the equivalents based on the loading of the solid phase could be 5 for the first 50 couplings and then reduced to 3 for the remaining synthesis. But also, other combinations and more steps of reduction throughout the synthesis can be applied.

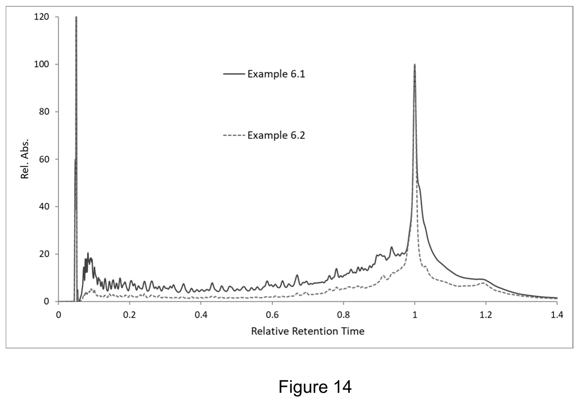

In a preferred embodiment the synthesis is carried out with an activator selected from the group of imidazoles, a capping reagent selected from acetanhydride in acetonitrile or N-Methylimidazole and 2,6-lutidine in acetonitrile, a detritylation time of less than 5 minutes and a capping equivalent of less than 10 Eq, acetanhydride (see. Example 6 and Figure 14).

The above steps are repeated as often as necessary until the desired amount of ribonucleotide units has been coupled to the solid support to obtain the desired oligonucleotide molecule.

The solid support is then (step I)) treated with a solution of an alkyl amine or a cyclic or a bicyclic amine to remove phosphorous protecting groups. Examples of alkyl amines to be used are methylamine, ethylamine, isopropylamine, diethylamine or trimethylamine. Examples of cyclic amines are pyrrolidine, piperidine, morpholine, or piperazine. An example for a bicyclic amine is1,8-Diazoabicyclo(5.4.0)undec-7-ene.

These compounds are used in an appropriate solvent which is preferably acetonitrile. Preferably a 20% (v/v) solution of diethylamine in acetonitrile is used.

The oligoribonucleotide compound is then deprotected and cleaved to obtain the crude oligonucleotide molecule (step n) which is optionally further purified. Basically, any known reagent for deprotecting oligoribonucleotides can be used. Examples are ammonia, a mixture of ammonia and aqueous methylamine (AMA), a mixture of hydrazine/ethanolamine and methanol or diethyl amine. Moreover, basically, any reagent for cleaving the oligoribonucleotide compound from the solid support can be used. Examples for agents to be used are ammonium hydroxide, diisopropyl amine or ethanolic ammonium hydroxide.

The deprotecting and cleaving step n) is preferably carried out as an in-column deprotection step and is preferably carried out with a solution of a basic alkylamine like methylamine, diethylenamine, tert-butylamine. More preferably, the basic alkylamine is methyl amine and, very preferably, a 40% aqueous methyl amine solution is used.

Preferably this step is carried out at a temperature ≤ 24°C.

For an oligonucleotide containing ribonucleotide monomers with 2'-silyl protecting groups an additional 2'-deprotection step is necessary within step n. The deprotection solution is filtered to remove the solid support and DMSO and buffered HF is added at < RT. 2'-deprotection is conducted for 1-8h at a temperature ≤ 65°C, more preferably at a temperature ≤45°C.

Between each of the above steps c-l, one or more washing steps can be carried out. These washing steps can be carried out with any washing solvent commonly used for solid-phase phosphoramidite-synthesis. Preferably, the solvent is acetonitrile.

Preferably, one or more porous spacer plate(s) is or are placed on the top of the solid support. Such spacer plates are commercially available. Said porous distributor or spacer plate has preferably a thickness of 0.2 to 10 cm, more preferably 1 to 3 cm. Preferably the spacer plate has a pore size from equal to or above 20 µm, more preferably equal to or above 80 µm.The above synthesis reaction can be carried out in the above order, or the order can be detritylation, coupling, thiolation / oxidation, capping or detritylation, coupling, capping, thiolation / oxidation or detritylation, coupling, capping, thiolation / oxidation, capping. This means that the capping reaction can either be performed before the thiolation / oxidation, after the thiolation / oxidation, or before and after the thiolation / oxidation. Furthermore, capping can be omitted totally or be applied at the same time as thiolation/oxidation.

In a preferred embodiment the synthesis is conducted at a temperature between 10 and 25°C, preferably 14 - 28°C, more preferably between 18 and 24°C, still more preferably between 15 and 20°C (see Example 10 and Figures 21 and 22). The very most preferred temperature is between 18 and 24°C.

The present invention, moreover, relates to a method for purifying an oligonucleotide molecule comprising 50 and 500 nucleotides, synthesized by solid-phase phosphoramidite-synthesis comprising at least the steps
a) providing a crude oligoribonucleotide solution obtained by solid-phase phosphoramidite-synthesis;
b) optionally desalting the crude oligoribonucleotide solution;
c) purifying the desalted oligoribonucleotide solution by a chromatographic method or a combination of chromatographic methods, whereby a buffer having a pH of 3 to 8.5 is used and the chromatographic method is selected from anion exchange chromatography, ion exchange purification, hydrophobic interaction chromatography and/or ion pair - reversed phase purification;
d) obtaining purified oligonucleotide molecule.
whereby the purification is carried out at a temperature between room temperature and 80°C.

Preferably, an oligonucleotide molecule synthesized by the above synthesis method is purified. For the purification method, step c) is important, i.e. a combination of chromatographic methods, whereby a buffer having a pH of 3 to 8.5 is used. Preferably, said buffer is an acetate buffer containing 5 to 30%, more preferably 10 to 20% of an organic solvent (e.g. acetonitrile, methanol, ethanol, propanol, 2-propanol or a mixture of these). As buffer salts also phosphate, citrate, tris(hydroxymethyl)aminomethane (TRIS)- or other suitable buffer salts can be used.

The chromatographic method is selected from anion exchange chromatography, ion exchange purification, hydrophobic interaction chromatography and/or ion pair - reversed phase purification or a combination thereof. These methods are generally known in the art and readily available to a person skilled in the art. The purification (p.Ex. anion exchange chromatography) is conducted at a temperature between room temperature and 80°C, preferably between 45°C and 80°C, more preferably between 50 and 80°C, even more preferably between 50°C and 70°C (including the border values).

Preferably the purification temperature at the column outlet is elevated to >50°. Preferably heated jacket and preheated solvents are used for the purification column.

In a preferred embodiment IP-RP purification is used as a second purification step after initial IEX purification or vice versa.

The above phosphoramidite-synthesis method of oligonucleotides is a robust and versatile method allowing for a precise control over the sequence composition and possible modification. The combination of the specific process features surprisingly allows the synthesis of long oligonucleotide molecules in high purity.

The major improvements of the various specific embodiments of the phosphoramidite-synthesis method of the invention are shown in the following Table 1:

**Table 1**

| **Process step** | **Standard process** | **Process of the invention** | **Impact on product quality/process** |
|---|---|---|---|
| Synthesis | Tetrazole-based activators (ETT, BTT, Activator 42) | Activator with a pKₐ between 5 and 5.5 | Impurity profile changed, late-eluting impurities which are difficult to remove are significantly reduced |
| | Single coupling for all nucleotides, amidite concentration 0.15 M | Double coupling for up to 50 bases, amidite concentration 0.2 M | Coupling yields higher for first bases, overall yield increased |
| | Coupling solution is directly applied to synthesis column | Porous spacer plate is inserted on top of the synthesis column | More homogeneous mixing of coupling mixture -> better coupling yield |
| | Detritylation with 3% DCA based on UV watch | a) Detritylation with fixed CV (contact time less than five minutes) of 3% DCA solution | Contact times shortened, less consumption of DCA, better product quality (less late-eluting impurities) |
| | | b) Detritylation with fixed CV (contact time less than five minutes) of 5% DCA solution | |
| | Capping with Ac₂O (26eq) | Reduced capping equivalents < 15 eq) | Higher crude yield, higher purity, less truncation products |
| | Synthesis at uncontrolled room temperature | Synthesis at temperature ≤24°C | Higher crude product purity |
| | | | |

The advantages of the process embodiments of the present invention as regards cleavage, deprotection and crude UF of standard processes are evident from the following Table 2:

**Table 2**

| **Standard process** | **Process of the invention** | **Impact on product quality/process** |
|---|---|---|
| I) Cleavage and deprotection in beaker or vessel with ammonia or methylamine | I) In-column deprotection with alkyl amine at ≤24°C | - Process is easily scalable to very large amounts without precipitation step |
| | II) Filtration and DMSO wash | - Mild conditions during desilylation improve product quality |
| II) filtration from solid support and wash with 30% ethanol | III) NEt₃x3HF added to solution for desilylation, incubation at 40°C | |
| III) Evaporation of ammonia/methylamine | | - Separation of shortmers through crude CF improves product quality before purification |
| IV) Desilylation with DMSO/NEt₃/NEt₃X3 HF, 3h, 65°C (slightly acidic pH) | | |
| | IV) Dilution with phosphate buffer | - Higher product yields through elimination of precipitation step |
| | V) Crude desalting against ≥2 kDa membrane | - Process is faster due to absence of precipitation step |
| V) Precipitation with Butanol | | |
| VI) Resuspension in buffer for purification, (no) crude UF | | |

The advantages of the claimed purification method (HPLC purification) in view of processes of the art are shown in the following Table 3:

**Table 3**

| **Standard process** | **Process of the invention** | **Impact on product quality/process** |
|---|---|---|
| Purification with AIEX chromatography, neutral buffer systems , purification at room temperature or at 40°C | a) Elevation of purification temperature:, column outlet >50°C | |
| | b) heated jacket and preheated solvents used for purification columns | - Achievable yields and final purities increased throughout all steps |
| | c) IP-RP purification as second purification step after initial IEX purification | - IP-RP purification can significantly improve impurity profile, later eluting impurities are significantly reduced |

The following examples serve to illustrate the subject-matter of the present invention:

### EXAMPLES

### Example 1:

Synthesis of a 100mer RNA/OMe oligonucleotide, comparison between a baseline protocol and a synthesis using an additional polyethylene porous spacer plate on an Åkta Oligopilot^{®} 100 synthesizer

### 1.1 Synthesis without porous spacer plate:

### 1.1.1 Synthesis

10.8 g of rU CPG were loaded into a Fineline 35 column (bed height: 4cm). The piston of the column was placed on top of the CPG.

Detritylation was performed by treating the solid support with a detritylation solution containing 3-5 % DCA in toluene for a fixed time within 2 - 7 minutes. The solid support was washed with acetonitrile after detritylation.

Coupling was achieved by treating the support with a mixture of a 0.15 M solution of 2'-TBDMS protected RNA amidites or 2'-OMe amidites in acetonitrile and a 0.5 M activator solution in acetonitrile as activating reagent in a volumetric mixture of > 50% activator solution and < 50% amidite solution. The coupling solution was recycled for 8 minutes through the column via a recirculation loop. The solid support was washed with acetonitrile after coupling.

Oxidation was achieved by treating the solid support with a 0.05 M solution of iodine in pyridine/water until oxidation was completed. For some cycles oxidation was replaced by thiolation. For thiolation the solid support was treated with e.g. 0.2 M PADS. After oxidation or thiolation the solid support was washed with acetonitrile

Unreacted 5'-hydroxyl groups were capped by treating the solid support with a 1:1 volumetric mixture (0.2 column volumes (CV) each ) of two solutions:
Capping solution 1: N-Methylimidazole/2,6-Lutidine/Acetonitrile
Capping solution 2: tert-Butylphenoxyacetic acid anhydride in acetonitrile

After capping the solid support was washed with acetonitrile.

Detritylation, coupling, oxidation or thiolation and capping were repeated for each nucleotide until the desired length (100mer) was achieved.

The phosphorous protecting groups were removed by treating the solid support on column with a solution of an alkyl amine in acetonitrile for 10 - 60 minutes.

### 1.1.2 Cleavage & Deprotection, Desalting

The solid support was washed with acetonitrile to remove alkyl amine solution. The solid support was dried under reduced pressure to remove remaining solvent and then treated with 40% aqueous methylamine solution for 1 - 8 hours to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-protecting groups. The oligonucleotide solution was diluted with buffer.

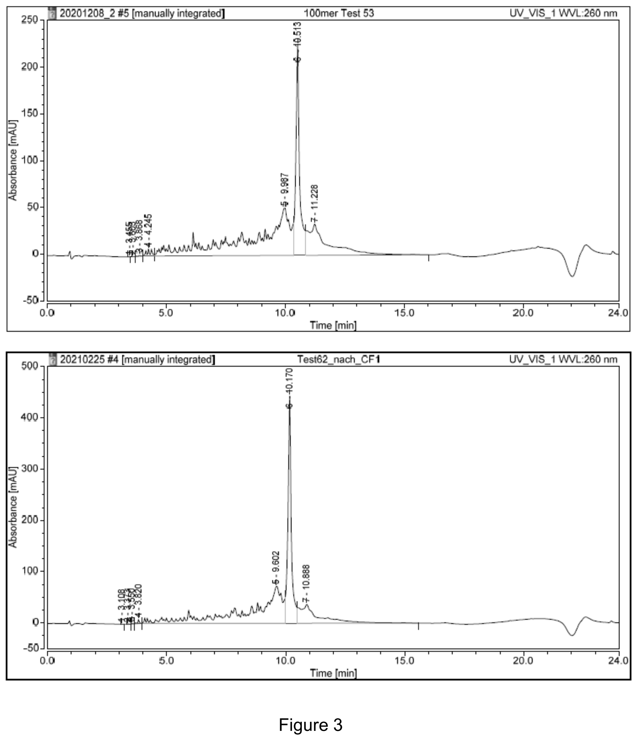

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via IP-HPLC and LC-MS analysis (see Figure 3 for purity analysis).

### 1.2 Synthesis with porous spacer plate:

10.8 g of rU CPG were loaded into a Fineline 35 column. On top of the solid support a porous spacer plate (same diameter as column, 1 cm height) was placed and the piston adjusted accordingly in such a way that the piston was directly on top of the porous spacer plate. The column volume was not adjusted in the synthesis method and kept constant in comparison to 1.1

All other steps were performed in the same way as described under 1.1.

Table 4 shows an overview of the results.

**Table 4: Overview of yield and purity data of synthesis 1.1 and 1.2**

| **Synthesis conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|
| No porous spacer plate (baseline) | 24.75 | - | 54.79 | 20.47 | 213 |
| With porous spacer plate | 33.71 | 8.96 | 49.95 | 16.33 | 217 |

### Example 2:

Synthesis of a 100mer RNA oligonucleotide, comparison between using different capping reagents and different equivalents of capping reagent

### 2.1 Capping reagents mix A:

### 2.1.1. Synthesis

11.0 g of rU CPG were loaded into a Fineline 35 column. On top of the solid support a porous spacer plate was placed as described in chapter 1.2. Synthesis steps were performed as described in chapter 1.1.

Unreacted 5'-hydroxyl groups were capped by treating the solid support with a 1:1 volumetric mixture (0.2 CV each) of two solutions:
Capping solution 1: *N*-Methylimidazole/2,6-Lutidine/Acetonitrile
Capping solution 2: *tert*-Butylphenoxyacetic acid anhydride in acetonitrile (< 5 eq. based on synthesis scale)

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 2.1.2 Cleavage & Deprotection, Desalting

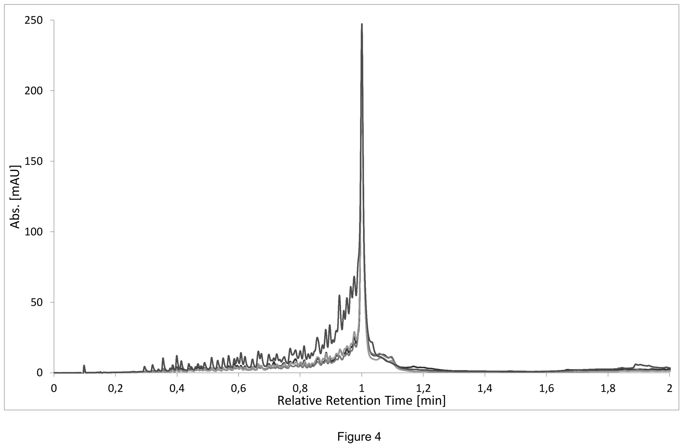

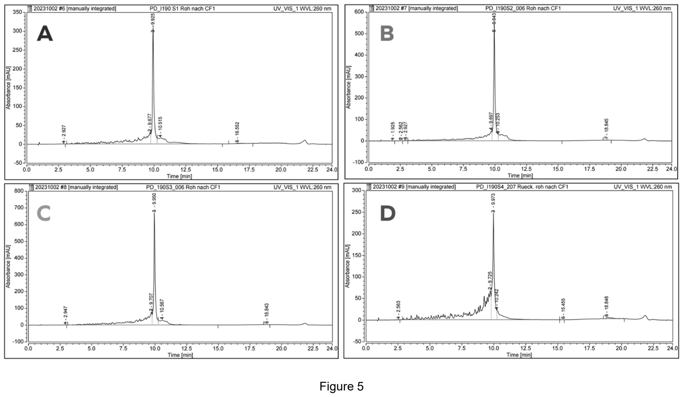

Cleavage, deprotection and desalting steps were performed as described in chapter 1.1. The purity and identity were determined via IP-HPLC and LC-MS analysis (see Figure 4 for comparison of IP-RP HPLC analyses of syntheses using capping reagent mix A-D, see Figure 5 A for individual analysis).

### 2.2 Capping reagents mix B:

### 2.2.1 Synthesis

11.0 g of rU CPG were loaded into a Fineline 35 column. On top of the solid support a porous spacer plate was placed as described in chapter 1.2. Synthesis steps were performed as described in chapter 1.1.

Unreacted 5'-hydroxyl groups were capped by treating the solid support with a 1:1 volumetric mixture (0.2 CV each ) of two solutions:
Capping solution 1: Acetic anhydride in acetonitrile (< 15 eq. based on synthesis scale)
Capping solution 2: *N*-Methylimidazole/2,6-Lutidine/Acetonitrile

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 2.2.2 Cleavage & Deprotection, Desalting

Cleavage, deprotection and desalting steps were performed as described in chapter 1.1. The purity and identity were determined via IP-HPLC and LC-MS analysis (see Figure 4 for comparison of IP-RP HPLC analyses of syntheses using capping reagent mix A-D, see Figure 5 B for individual analysis).

### 2.3 Capping reagents mix C:

### 2.3.1 Synthesis

11.0 g of rU CPG were loaded into a Fineline^{®} 35 column. On top of the solid support a porous spacer plate was placed as described in chapter 1.2. Synthesis steps were performed as described in chapter 1.1.

Unreacted 5'-hydroxyl groups were capped by treating the solid support with a 1:1 volumetric mixture (0.2 CV each ) of two solutions:
Capping solution 1: *N*-Methylimidazole/2,6-Lutidine/Acetonitrile
Capping solution 2: Isobutyric anhydride in acetonitrile (< 15eq. based on synthesis scale)

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 2.3.2 Cleavage & Deprotection, Desalting

Cleavage, deprotection and desalting steps were performed as described in chapter 1.1. The purity and identity were determined via IP-HPLC and LC-MS analysis (see Figure 4 for comparison of IP-RP HPLC analyses of syntheses using capping reagent mix A-D, see Figure 5 C for individual analysis).

### 2.4 Capping reagents mix D (standard capping conditions):

### 2.4.1 Synthesis

11.0 g of rU CPG were loaded into a Fineline^{®} 35 column. On top of the solid support a porous plate was placed as described in chapter 1.2. Synthesis steps were performed as described in chapter 1.1.

Unreacted 5'-hydroxyl groups were capped by treating the solid support with a 1:1 volumetric mixture (0.2 CV each ) of two solutions:
Capping solution 1: Acetic anhydride in acetonitrile (> 40 eq. based on synthesis scale)
Capping solution 2: *N*-Methylimidazole/2,6-Lutidine/Acetonitrile

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 2.4.2 Cleavage & Deprotection, Desalting

Cleavage, deprotection and desalting steps were performed as described in chapter 1.1. The purity and identity were determined via IP-HPLC and LC-MS analysis (see Figure 4 for comparison of IP-RP HPLC analyses of syntheses using capping reagent mix A-D, see Figure 5 D for individual analysis).

Table 5 shows an overview of yield and purity data of the above synthesis.

**Table 5: Overview of yield and purity data of synthesis 2.1, 2.2, 2.3 and 2.4**

| **Synthesis** | **Capping procedure** | **FLP Purity [%, UV]** | **FLP purity increase compare d to baseline [%, UV]** | **Early-eluting impurities [%, UV]** | **Late-eluting impurities [%, UV]** | **Yield [OD/µmol]** |
|---|---|---|---|---|---|---|
| 2.1 | Tac₂O (<5eq) | 42.72 | 12.19 | 39.88 | 17.41 | 199 |
| 2.2 | Ac₂O (<15 eq) | 48.17 | 17.64 | 36.00 | 15.83 | 228 |
| 2.3 | iBu₂O (< 15eq) | 43.34 | 12.81 | 45.26 | 11.40 | 246 |
| 2.4 | Baseline (Ac₂O >40eq) | 30.53 | - | 59.78 | 9.69 | 219 |

### Example 3

Synthesis of a 100mer RNA/OMe oligonucleotide, comparison of different contact times of detritylation reagent

### 3.1 Contact time of detritylation reagent with solid support ->5 minutes:

### 3.1.1 Synthesis

11.0 g of rU CPG were loaded into a Fineline^{®} 35 column. Synthesis steps were performed as described in chapter 1.1.

Detritylation was performed by treating the solid support with a detritylation solution containing 3-5 % DCA in toluene for a fixed time >5 minutes. The solid support was washed with acetonitrile after detritylation.

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 3.1.2 Cleavage & Deprotection, Desalting

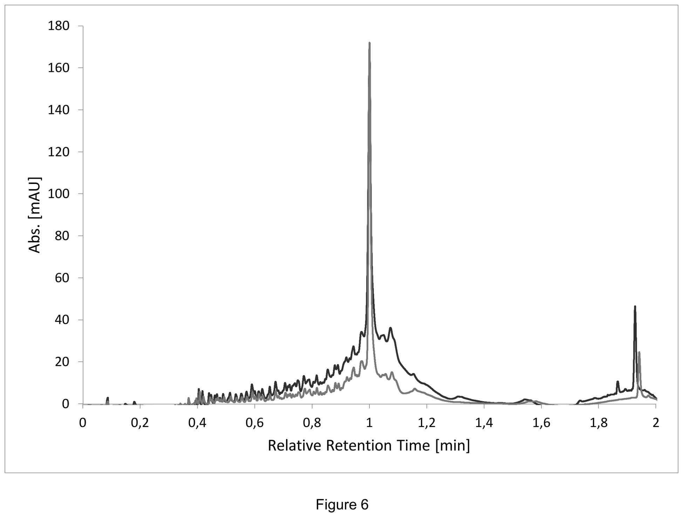

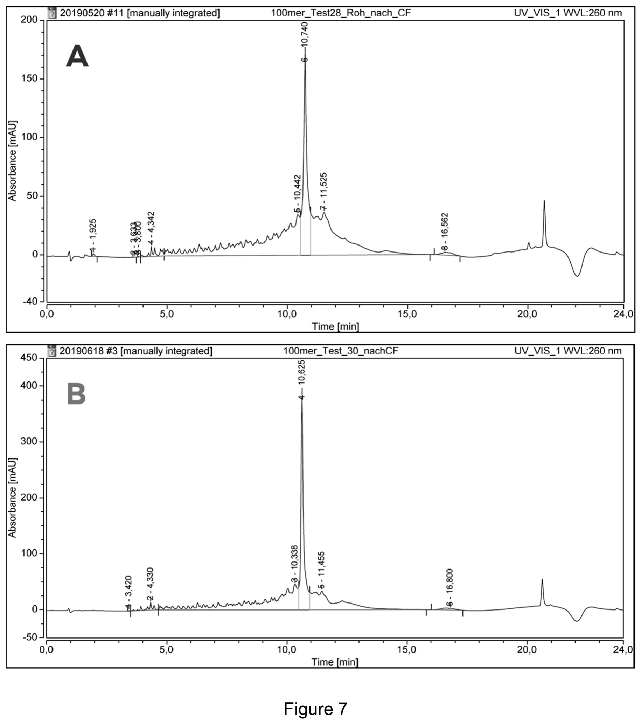

Cleavage, deprotection and desalting steps were performed as described in chapter 1.1. The purity and identity were determined via IP-HPLC and LC-MS analysis (see Figure 6 for comparison of IP-RP HPLC analyses of syntheses 3.1 and 3.2, see Figure 7 for individual analysis).

### 3.2 Contact time of detritylation reagent with solid support <5 minutes:

### 3.2.1 Synthesis

11.0 g of rU CPG were loaded into a Fineline^{®} 35 column. Synthesis steps were performed as described in chapter 1.1.

Detritylation was performed by treating the solid support with a detritylation solution containing 3-5 % DCA in toluene for a fixed time < 5 minutes The solid support was washed with acetonitrile after detritylation.

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 3.2.2 Cleavage & Deprotection, Desalting

Cleavage, deprotection and desalting steps were performed as described in chapter 1.1. The purity and identity were determined via IP-HPLC and LC-MS analysis (see Figure 5 for comparison of IP-RP HPLC analyses of syntheses 3.1 and 3.2, see Figure 6 for individual analysis).

Table 6 shows an overview of yield and purity data of the above synthesis

**Table 6: Overview of yield and purity data of synthesis 2.1, 2.2, 2.3 and 2.4**

| **Synthesis** | **Detritylation conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|---|
| 3.1 | Baseline (> 5min) | 21.82 | - | 43.00 | 35.16 | 268 |
| 3.2 | Short (< 5 min) | 30.49 | 8.67 | 43.01 | 26.51 | 247 |

### Example 4

Synthesis and purification of a 100mer RNA/OMe oligonucleotide, comparison of different purification temperatures

### 4.1 Synthesis

11 g of rU CPG were loaded into a Fineline^{®} 35 column. The piston of the column was placed on top of the CPG.

Detritylation was performed by treating the solid support with a detritylation solution containing 3-5 % DCA in toluene for a fixed time < 5 minutes. The solid support was washed with acetonitrile after detritylation.

Coupling was achieved by treating the support with a mixture of a 0.15 M solution of 2'-TBDMS protected RNA amidites or 2'-OMe amidites in acetonitrile and a 0.5 M activator solution in acetonitrile as activating reagent in a volumetric mixture of > 50% activator solution and < 50% amidite solution. The coupling solution was recycled for 8 minutes through the column via a recirculation loop. The solid support was washed with acetonitrile after coupling.

Oxidation was achieved by treating the solid support with a 0.05 M solution of iodine in Pyridine/water until oxidation was completed. For some cycles oxidation was replaced by thiolation. For thiolation the solid support was treated with e.g. 0.2 M PADS. After oxidation or thiolation the solid support was washed with acetonitrile.

Unreacted 5'-hydroxyl groups were capped by treating the solid support with a 1:1 volumetric mixture (0.2 CV each ) of two solutions:
Capping solution 1: *N*-Methylimidazole/2,6-Lutidine/Acetonitrile
Capping solution 2: tert-Butylphenoxyacetic acid anhydride in acetonitrile

After capping the solid support was washed with acetonitrile.

Detritylation, coupling, oxidation or thiolation and capping were repeated for each nucleotide until the desired length (100mer) was achieved.

The phosphorous protecting groups were removed by treating the solid support on column with a solution of an alkyl amine in acetonitrile for 10 - 60 minutes.

### 4.2 Cleavage & Deprotection, Desalting

The solid support was washed with acetonitrile to remove alkyl amine solution. The solid support was dried under reduced pressure to remove remaining solvent and then treated with 40 % aqueous methylamine solution for 1 - 8 hours to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via IP-HPLC and LC-MS analysis (see Figure 5 for purity analysis). The crude product was split into three equal portions afterwards and purified at different temperatures via AIEX chromatography.

Table 7 shows an overview of yield and purity data of the above synthesis.

**Table 7: Overview of yield and purity data of synthesis 4.1**

| **FLP Purity [%, UV]** | **Early-eluting impurities [%, UV]** | **Late-eluting impurities [%, UV]** |
|---|---|---|
| 37.34 | 40.32 | 22.34 |

### 4.3.1 Purification at ambient temperature

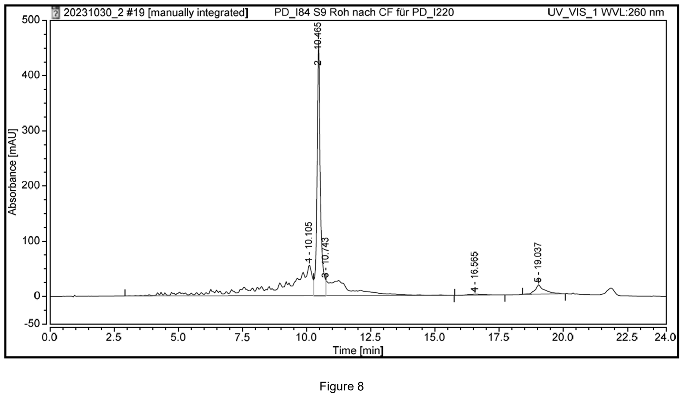

The oligonucleotide was purified via strong anion exchange chromatography using an aqueous phosphate buffer containing 10-20 % organic solvent (buffer A) and the same buffer containing 0.5-2 M sodium chloride or sodium bromide (buffer B). Crude oligonucleotide solution (10-20 mg/mL column volume) was loaded onto the column. After loading the oligonucleotide solution, the column was equilibrated with two column volumes of buffer A. The product was eluted from the column with a two-step linear gradient. Fractions were collected automatically based on UV absorbance. The fraction volume was set to ¼ of the total column volume. After elution of the product the column was washed with one column volume of buffer B and then reequilibrated to buffer A. The purification profile is shown in Figure 8.

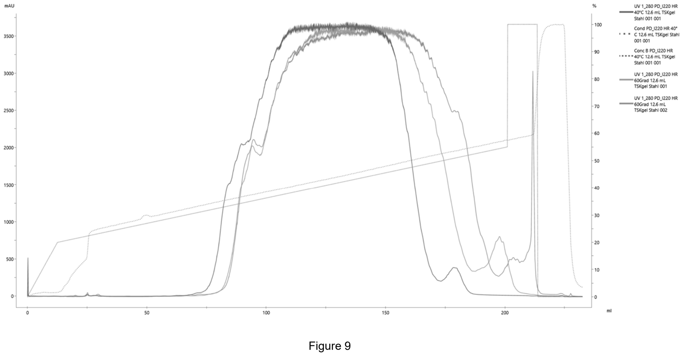

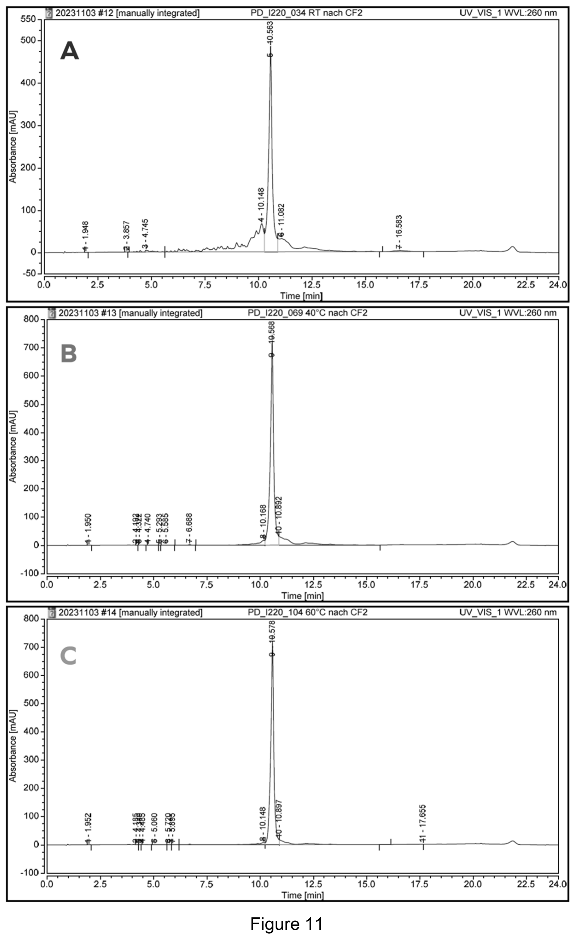

Selected fractions were analyzed via IP-HPLC and the purest fraction were pooled and desalted. The desalted pool was analyzed via IP-HPLC (see Figure 9 for comparison of IP-RP HPLC analyses of synthesis after purification using different temperature, see Figure 11 A for individual analysis)

### 4.3.2 Purification at elevated temperature (<45°C)

The purification was done in the same way as described in 4.3.1 (same gradient, same product loading, same buffer systems). In contrast to 4.3.1 the purification buffer as well as the column itself were heated to 35-45°C

The purification profile is shown in Figure 9.

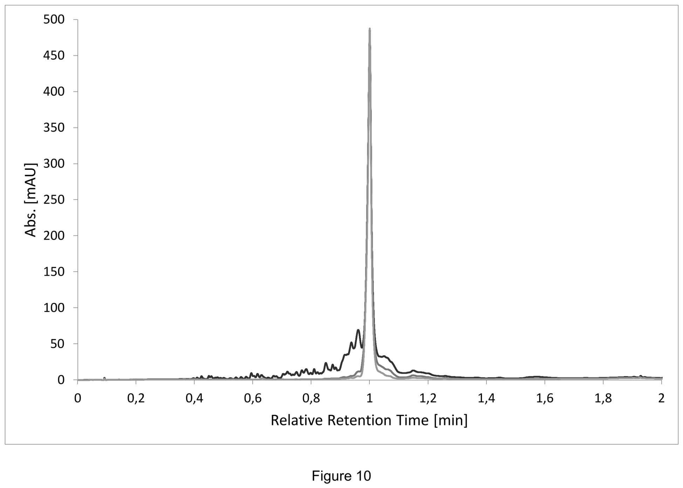

Selected fractions were analyzed via IP-HPLC and the purest fractions were pooled and desalted. The desalted pool was analyzed via IP-HPLC (see Figure 10 for comparison of IP-RP HPLC analyses of syntheses after purification using temperature 4.3.2, see Figure 11 B for individual analysis).

### 4.3.3 Purification at high temperature (>45°C)

The purification was done in the same way as described in 4.3.1 (same gradient, same product loading, same buffer systems). In contrast to 4.3.1 the purification buffer as well as the column itself were heated to 55-70°C as described in 4.3.2. The purification profile is shown in Figure 9.

Selected fractions were analyzed via IP-HPLC and the purest fractions were pooled and desalted. The desalted pool was analyzed via IP-HPLC (see Figure 10 for comparison of IP-RP HPLC analyses of syntheses after purification using temperature 4.3.3, see Figure 11 C for individual analysis).

Table 8 shows an overview of yield and purity data of the above synthesis.

**Table 8: Overview of yield and purity data after purification of synthesis 4.1**

| **Purification** | **Purification conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|---|
| 4.3.1 | Baseline (ambient temperature) | 50.78 | - | 31.58 | 17.64 | 24 |
| 4.3.2 | Elevated temperature (<45°C) | 81.48 | 30.70 | 4.87 | 13.63 | 41 |
| 4.3.3 | High temperature (>45°C) | 90.15 | 39.37 | 2.73 | 7.11 | 42 |

The above examples show that by applying the process conditions of the examples, RNA oligonucleotides having a high purity in a high yield can be obtained. The purities (% UV method) 2, 4, 3, 5 can reach values over 90%. Moreover, the yield in g/mMol can be as high as 2.85.

### Example 5

Synthesis of a 145mer RNA/OMe oligonucleotide, comparison between a baseline protocol with polyethylene porous spacer plate and a synthesis using additional native controlled pore glass (CPG) on polyethylene porous spacer plate on top of the solid support on an Åkta Oligopilot^{®} 100 synthesizer.

### 5.1 Synthesis without capped CPG:

### 5.1.1 Synthesis

6.48 g of 2'OMe rU CPG were loaded into a 2.7 x 5 cm steel column (bed height of loaded CPG: 4 cm). The polyethylene porous spacer plate was placed on top of the CPG.

Detritylation was performed by treating the solid support with a detritylation solution containing 3-5 % DCA in toluene for a fixed time within 2 - 7 minutes. The solid support was washed with acetonitrile after detritylation.

Coupling was achieved by treating the support with a mixture of a 0.20 M solution of 2'-TBDMS protected RNA amidites or 2'-OMe amidites in acetonitrile and a 0.5 M activator solution in acetonitrile as activating reagent in a volumetric mixture of > 50% activator solution and < 50% amidite solution. The coupling solution was recycled for 8 minutes through the column via a recirculation loop. The solid support was washed with acetonitrile after coupling.

Oxidation was achieved by treating the solid support with a 0.05 M solution of iodine in pyridine/water until oxidation was completed. For some cycles oxidation was replaced by thiolation. For thiolation the solid support was treated with e.g. 0.2 M PADS. After oxidation or thiolation the solid support was washed with acetonitrile

Unreacted 5'-hydroxyl groups were capped by treating the solid support with a 1:1 volumetric mixture (0.2 column volumes (CV) eachr) of two solutions:
Capping solution 1: N-Methylimidazole/2,6-Lutidine/Acetonitrile
Capping solution 2: tert-Butylphenoxyacetic acid anhydride in acetonitrile

After capping the solid support was washed with acetonitrile.

Detritylation, coupling, oxidation or thiolation and capping were repeated for each nucleotide until the desired length (145mer) was achieved.

The phosphorous protecting groups were removed by treating the solid support on column with a solution of an alkyl amine in acetonitrile for 10 - 60 minutes.

### 5.1.2 Cleavage & Deprotection, Desalting

The solid support was washed with acetonitrile to remove alkyl amine solution. The solid support was dried under reduced pressure to remove remaining solvent and then treated with 40% aqueous methylamine solution (wt. %) for 1 - 8 hours to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-TBDMS protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via high resolving IP-HPLC and LC-MS analysis (see Figure 13 for IP-HPLC analysis).

### 5.2 Synthesis using native CPG on top of porous spacer plate:

6.76 g of 2'OMe rU CPG were loaded into a 2.7 x 7 cm steel column. The polyethylene porous spacer plate was placed on top of the CPG and the column was backfilled with the native CPG (bed height of loaded CPG: 4 cm).

All other steps were performed in the same way as described under 5.1.

Table 9 shows an overview of the results.

**Table 9: Overview of yield and purity data of synthesis 5.1 and 5.2**

| **Synthesis conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|
| No capped native CPG (baseline) | 16.21 | - | 71.50 | 12.29 | 238 |
| With capped native CPG | 24.62 | 8.41 | 53.69 | 21.70 | 329 |

### Example 6:

Synthesis of a 130mer RNA/OMe oligonucleotide, comparison of different synthesis parameters on an Åkta Oligopilot^{®} 100 synthesizer.

### 6.1 Synthesis using acidic activator with pKₐ < 5, detritylation time > 5 min and > 15 eq Ac₂O

### 6.1.1 Synthesis

### 6.91 g of 2'OMe rU CPG were loaded into a 2.7 x 4 cm steel column (bed height: 4 cm).

Detritylation was performed by treating the solid support with a detritylation solution containing 3-5 % DCA in toluene for a fixed time > 5 minutes. The solid support was washed with acetonitrile after detritylation.

Coupling was achieved by treating the support with a mixture of a 0.20 M solution of 2'-TBDMS protected RNA amidites or 2'-OMe amidites in acetonitrile and a acidic activator with pKₐ < 5 as activating reagent in a volumetric mixture of > 50% activator solution and < 50% amidite solution. The coupling solution was recycled for 8 minutes through the column via a recirculation loop. The solid support was washed with acetonitrile after coupling.

Oxidation was achieved by treating the solid support with a 0.05 M solution of iodine in pyridine/water until oxidation was completed. For some cycles oxidation was replaced by thiolation. For thiolation the solid support was treated with e.g. 0.2 M PADS. After oxidation or thiolation the solid support was washed with acetonitrile

Unreacted 5'-hydroxyl groups were capped by treating the solid support with a 1:1 volumetric mixture (0.2 column volumes (CV) each ) of two solutions to apply > 15 eq of Ac₂O:
Capping solution 1: Ac₂O in Acetonitrile
Capping solution 2: N-Methylimidazole and 2,6-lutidine in acetonitrile

After capping the solid support was washed with acetonitrile.

Detritylation, coupling, oxidation or thiolation and capping were repeated for each nucleotide until the desired length (130mer) was achieved.

The phosphorous protecting groups were removed by treating the solid support on column with a solution of an alkyl amine in acetonitrile for 10 - 60 minutes.

### 6.1.2 Cleavage & Deprotection, Desalting

Cleavage, deprotection and desalting were performed as described in 1.1. The purity and identity were determined via high resolving IP-HPLC and LC-MS analysis (see Figure 14 for IP-HPLC analysis).

### 6.2 Synthesis using activator from the group of imidazoles, detritylation time < 5 min and < 10 eq Ac₂O

### 6.2.1 Synthesis

### 6.91 g of 2'OMe rU CPG were loaded into a 2.7 x 4 cm steel column (bed height: 4 cm).

Detritylation was performed by treating the solid support with a detritylation solution containing 3-5 % DCA in toluene for a fixed time < 5 minutes. The solid support was washed with acetonitrile after detritylation.

Coupling was achieved by treating the support with a mixture of a 0.20 M solution of 2'-TBDMS protected RNA amidites or 2'-OMe amidites in acetonitrile and an activator from the group of imidazoles in a volumetric mixture of > 50% activator solution and < 50% amidite solution. The coupling solution was recycled for 8 minutes through the column via a recirculation loop. The solid support was washed with acetonitrile after coupling.

Oxidation was achieved as described in 6.1

Unreacted 5'-hydroxyl groups were capped by treating the solid support with a 1:1 volumetric mixture (0.2 column volumes (CV) each ) of two solutions described in 6.1 to apply < 10 eq of Ac₂O:

### 1.1.2 Cleavage & Deprotection, Desalting

Cleavage, deprotection and desalting were performed as described in 5.1. The purity and identity were determined via high resolving IP-HPLC and LC-MS analysis (see Figure 14 for IP-HPLC analysis).

Table 10 shows an overview of the results.

**Table 10: Overview of yield and purity data of synthesis 2.1 and 2.2**

| **Synthesis conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|
| activator with pKₐ < 5, detritylation time > 5 min and >15 eq Ac₂O | 8.75 | - | 63.33 | 27.92 | 287 |
| activator from group of imidazoles, detritylation time < 5 min and < 10 eq Ac₂O | 19.04 | 10.29 | 50.73 | 30.23 | 335 |

### Example 7:

Synthesis of a 100mer RNA/OMe oligonucleotide, comparison of synthesis quality after carrying out coupling twice within a single cycle for 0,5 and 50 nucleotides from 3'-end

### 7.1 Baseline synthesis without double coupling

### 7.1.1. Synthesis

7.17 g of rU CPG were loaded into a 2.7 x 4 cm steel column (bed height: 4 cm). On top of the solid support a porous spacer plate was placed as described in chapter 5.1. Synthesis steps were performed as described in chapter 5.1.

### 7.1.2 Cleavage & Deprotection, Desalting

Cleavage, deprotection and desalting steps were performed as described in chapter 5.1. The purity and identity were determined via high resolving IP-HPLC and LC-MS analysis (see Figure 15 for comparison of IP-RP HPLC analyses of syntheses 7.1-7.3, see Figure 16 A for individual analysis).

### 7.2 Synthesis using 5 phosphoramidite double couplings at 3' end

### 7.2.1 Synthesis

7.17 g of rU CPG were loaded into a 2.7 x 5 cm steel column (bed height: 4 cm). On top of the solid support a porous spacer plate was placed as described in chapter 5.1. Synthesis steps were performed as described in chapter 5.1 except during the first 5 phosphoramidite coupling cycles where coupling was performed twice. The coupling solution was recycled for 8 minutes through the column via a recirculation loop and the solid support was then washed with acetonitrile. The process was subsequently repeated before the oxidation/thiolation cycle was initiated.

### 7.2.2 Cleavage & Deprotection, Desalting

Cleavage, deprotection and desalting steps were performed as described in chapter 5.1. The purity and identity were determined via high resolving IP-HPLC and LC-MS analysis (see Figure 15 for comparison of high resolving IP-RP HPLC analyses of syntheses 7.1-7.3, see Figure 16 B for individual analysis).

### 7.3 Synthesis using 50 phosphoramidite double couplings at 3' end

### 7.3.1 Synthesis

7.23 g of rU CPG were loaded into a 2.7 x 45 cm steel column (bed height: 4 cm). On top of the solid support a porous spacer plate was placed as described in chapter 5.1. Synthesis steps were performed as described in chapter 5.1 except during the first 50 phosphoramidite coupling cycles where coupling was performed twice. The coupling solution was recycled for 8 minutes through the column via a recirculation loop and the solid support was then washed with acetonitrile. The process was subsequently repeated before the oxidation/thiolation cycle was initiated.

### 7.3.2 Cleavage & Deprotection, Desalting

Cleavage, deprotection and desalting steps were performed as described in chapter 5.1. The purity and identity were determined via high resolving IP-HPLC and LC-MS analysis (see Figure 15 for comparison of IP-RP HPLC analyses of syntheses 7.1-7.3, see Figure 16 C for individual analysis).

**Table 11: Overview of yield and purity data of synthesis 7.1, 7.2 and 7.3**

| **Synthesis conditions** | **FLP Purity [%, UV]** | **FLP purity increase compared to baseline [%, UV]** | **Early-eluting impurities [%, UV]** | **Late-eluting impurities [%, UV]** | **Yield [OD/µmol]** |
|---|---|---|---|---|---|
| No double coupling | 32.96 | - | 51.12 | 15.93 | 205 |
| Double coupling of first 5 phosphoramidites | 37.17 | 4.21 | 46.96 | 15.87 | 215 |
| Double coupling of first 50 phosphoramidites | 37.16 | 4.20 | 47.24 | 15.60 | 239 |

### Example 8:

Synthesis of a 100mer RNA/OMe oligonucleotide, comparison between using different equivalents of capping reagent *tert*-Butylphenoxyacetic acid anhydride (Tac₂O).

### 8.1 Capping reagents mix A:

### 8.1.1. Synthesis

7.00 g of rU CPG were loaded into a 2.7 x 5 cm steel column. On top of the solid support a porous spacer plate was placed (bed height: 4 cm) as described in chapter 5.1. Synthesis steps were performed as described in chapter 5.1 except during the first 3 phosphoramidite coupling cycles. The coupling solution was recycled for 8 minutes through the column via a recirculation loop and the solid support was then washed with acetonitrile. The process was subsequently repeated before the oxidation/thiolation cycle was initiated.

Unreacted 5'-hydroxyl groups were capped by treating the solid support with a 1:1 volumetric mixture (0.2 CV each) of two solutions:
Capping solution 1: N-Methylimidazole/2,6-Lutidine/Acetonitrile
Capping solution 2: tert-Butylphenoxyacetic acid anhydride in acetonitrile 50-100 g/L (< 5 eq. based on synthesis scale)

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 8.1.2 Cleavage & Deprotection, Desalting

Cleavage, deprotection and desalting steps were performed as described in chapter 5.1. The purity and identity were determined via high resolving IP-HPLC and LC-MS analysis (see Figure 17 for comparison of IP-RP HPLC analyses of syntheses 8.1-8.3 using capping reagent mix A-C, see Figure 18 A for individual analysis).

### 8.2 Capping reagents mix B:

### 8.2.1 Synthesis

7.20 g of rU CPG were loaded into a 2.7 x 5 cm steel column. On top of the solid support a porous spacer plate was placed (bed height: 4 cm) as described in chapter 5.1. Synthesis steps were performed as described in chapter 5.1 except during the first 3 phosphoramidite coupling cycles. The coupling solution was recycled for 8 minutes through the column via a recirculation loop and the solid support was then washed with acetonitrile. The process was subsequently repeated before the oxidation/thiolation cycle was initiated.

Unreacted 5'-hydroxyl groups were capped by treating the solid support with a 1:1 volumetric mixture (0.2 CV each ) of two solutions:
Capping solution 1: N-Methylimidazole/2,6-Lutidine/Acetonitrile
Capping solution 2: tert-Butylphenoxyacetic acid anhydride in acetonitrile 150-240 g/L (< 10 eq. based on synthesis scale)

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 8.2.2 Cleavage & Deprotection, Desalting

Cleavage, deprotection and desalting steps were performed as described in chapter 5.1. The purity and identity were determined via IP-HPLC and LC-MS analysis (see Figure 17 for comparison of high resolving IP-RP HPLC analyses of syntheses 8.1-8.3 using capping reagent mix A-C, see Figure 18 B for individual analysis).

### 8.3 Capping reagents mix C:

### 8.3.1 Synthesis

7.00 g of rU CPG were loaded into a 2.7 x 5 cm steel column. On top of the solid support a porous spacer plate was placed (bed height: 4 cm) as described in chapter 5.1. Synthesis steps were performed as described in chapter 5.1 except during the first 3 phosphoramidite coupling cycles. The coupling solution was recycled for 8 minutes through the column via a recirculation loop and the solid support was then washed with acetonitrile. The process was subsequently repeated before the oxidation/thiolation cycle was initiated.

Unreacted 5'-hydroxyl groups were capped by treating the solid support with a 1:1 volumetric mixture (0.2 CV each ) of two solutions:
Capping solution 1: N-Methylimidazole/2,6-Lutidine/Acetonitrile
Capping solution 2: tert-Butylphenoxyacetic acid anhydride in acetonitrile 10-40 g/L (< 2 eq. based on synthesis scale)

Detritylation, coupling, oxidation and capping were repeated for each nucleotide until the desired length (100mer) was achieved.

The phosphorous protecting groups were removed by treating the solid support on column with a solution of an alkyl amine in acetonitrile for 10 - 60 minutes.

### 8.3.2 Cleavage & Deprotection, Desalting

Cleavage, deprotection and desalting steps were performed as described in chapter 5.1. The purity and identity were determined via high resolving IP-HPLC and LC-MS analysis (see Figure 17 for comparison of IP-RP HPLC analyses of syntheses 8.1-8.3 using capping reagent mix A-C, see Figure 18 C for individual analysis).

**Table 12: Overview of yield and purity data of synthesis 8.1, 8.2 and 8.3**

| **Synthesis** | **Capping procedure** | **FLP Purity [%, UV]** | **FLP purity increase compare d to baseline [%, UV]** | **Early-eluting impurities [%, UV]** | **Late-eluting impurities [%, UV]** | **Yield [OD/µmol]** |
|---|---|---|---|---|---|---|
| 8.1 | Tac₂O (<5 eq) | 30.75 | - | 49.12 | 20.13 | 210 |
| 8.2 | Tac₂O (<10 eq) | 19.96 | -10.79 | 63.69 | 16.35 | 178 |
| 8.3 | Tac₂O (<2 eq) | 34.83 | 4.08 | 47.55 | 17.63 | 240 |

### Example 9

Synthesis of a 100mer RNA/OMe oligonucleotide, comparison of different cleavage and deprotection (C&D) conditions.

### 9.1 Synthesis

### 6.93 g of rU CPG were loaded into a 2.7 x 5 cm steel column (bed height: 4 cm). The polyethylene porous spacer plate was placed on top of the CPG.

Synthesis steps were performed as described in chapter 5.1 except during the first 3 phosphoramidite coupling cycles where coupling was performed twice. The coupling solution was recycled for 8 minutes through the column via a recirculation loop and the solid support was then washed with acetonitrile. The process was subsequently repeated before the oxidation/thiolation cycle was initiated.

Detritylation, coupling, oxidation or thiolation and capping were repeated until the desired length (100mer) was achieved.

The phosphorous protecting groups were removed by treating the solid support on column with a solution of an alkyl amine in acetonitrile for 10 - 60 minutes.

### 9.2 Cleavage & Deprotection, Desalting

The solid support was washed with acetonitrile to remove alkyl amine solution. The solid support was dried under reduced pressure to remove remaining solvent divided into four equal aliquots and cleaved and deprotected according to the following conditions:

### 9.2.1 Cleavage & deprotection, desalting conditions A

The solid support after synthesis was treated with 40% aqueous methylamine solution (wt. %) for 1 - 8 hours at < 30 °C to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-TBDMS protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via high resolving IP-HPLC and LC-MS analysis (see Figure 19 for comparison of IP-RP HPLC analyses of C&D test 9.2.1-9.2.3 using conditions A-C, see Figure 20 A for individual analysis).

### 9.2.2 Cleavage & deprotection, desalting conditions B

The solid support after synthesis was treated with 40% aqueous methylamine solution (wt. %) for 1 - 8 hours at >30 °C to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-TBDMS protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via high resolving IP-HPLC and LC-MS analysis (see Figure 19 for comparison of IP-RP HPLC analyses of C&D test 9.2.1-9.2.3 using conditions A-C, see Figure 20 B for individual analysis).

### 9.2.3 Cleavage & deprotection, desalting conditions C

The solid support after synthesis was treated with 40% aqueous methylamine solution (wt. %) for >8 hours at < 30 °C to remove the base protecting groups and cleave the oligonucleotide from the solid support. The oligonucleotide solution was filtered to remove the solid support. Afterwards buffered HF was added at < RT. The reaction mixture was incubated for 1-8 h to remove the 2'-TBDMS protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via high resolving IP-HPLC and LC-MS analysis (see Figure 19 for comparison of IP-RP HPLC analyses of C&D test 9.2.1-9.2.3 using conditions A-C, see Figure 20 C for individual analysis).

### 9.2.4 Cleavage & deprotection, desalting conditions D

The solid support after synthesis was treated with 30-33 % ammonia solution (wt. %) for > 10 hours at > 40 °C to remove the base protecting groups and cleave the oligonucleotide from the solid support. The oligonucleotide solution was filtered to remove the solid support and ammonia was removed. Afterwards buffered HF was added at < RT. The reaction mixture was incubated for 1-8 h to remove the 2'-TBDMS protecting groups. The oligonucleotide solution was diluted with buffer.

The solution was pumped into an ultrafiltration device and the solvent was exchanged against water. The crude oligonucleotide solution was pumped out of the ultrafiltration device and the concentration was measured via UV absorbance on a photometer to determine yield. The purity and identity were determined via high resolving IP-HPLC and LC-MS analysis (see Figure 20 D for individual analysis).

Table 13 shows an overview of the results.

**Table 13: Overview of yield and purity data o C&D tests 9.2.1 - 9.2.4**

| **C&D conditions** | **FLP Purity [%, UV]** | **FLP purity decrease compared to baseline [%, UV]** | **Early-eluting impurities [%, UV]** | **Late-eluting impurities [%, UV]** | **Yield [OD/µmol]** |
|---|---|---|---|---|---|
| 1-8 h methylamine at < 30 °C, 1-8 h desilylation | 32.19 | - | 50.18 | 17.63 | 180 |
| 1-8 h methylamine at >30 °C, 1-8 h desilylation | 21.05 | 11.14 | 64.77 | 14.18 | 178 |
| >8 h methylamine at < 30 °C, 1-8 h desilylation | 17.27 | 14.92 | 70.70 | 12.03 | 211 |
| >10 h ammonia solution at >40- °C, 1-8 h desilylation | 2.79 | 29.40 | 93.03 | 4.18 | 137 |

### Example 10:

Synthesis of a 100mer RNA/OMe oligonucleotide, comparison of crude quality after synthesis at different temperatures on an Åkta Oligopilot^{®} 100 synthesizer.

### 10.1 Synthesis at <18 °C on steel-column:

### 10.1.1 Synthesis

8.35 g of rU CPG were loaded into a 2.7 x 5 cm steel column. On top of the solid support a porous spacer plate was placed (bed height: 4 cm) as described in chapter 5.1. The column was coated with flexible tubing and connected to a cryostat to ensure temperature control. The actual temperature was continuously monitored.

Detritylation was performed as described in chapter 5.1.

Coupling was achieved by treating the support with a mixture of a 0.15 M solution of 2'-TBDMS protected RNA amidites or 2'-OMe amidites in acetonitrile and a 0.5 M activator solution in acetonitrile as activating reagent in a volumetric mixture of > 50% activator solution and < 50% amidite solution. The coupling solution was recycled for 8 minutes through the column via a recirculation loop. The solid support was washed with acetonitrile after coupling.

Detritylation, oxidation or thiolation and capping were repeated for each nucleotide until the desired length (100mer) was achieved as described in chapter 5.1.

The phosphorous protecting groups were removed by treating the solid support on column with a solution of an alkyl amine in acetonitrile for 10 - 60 minutes.

### 10.1.2 Cleavage & Deprotection, Desalting

Cleavage, deprotection and desalting were performed as described in 5.1. The purity and identity were determined via IP-HPLC and LC-MS analysis (see Figure 21 for comparison of IP-RP HPLC analyses of temperature test 10.1-10.3, see Figure 21 A for individual analysis).

### 10.2 Synthesis between 18-24°C on steel-column:

### 10.2.1 Synthesis

8.45 g of rU CPG were loaded into a 2.7 x 5 cm steel column. On top of the solid support a porous spacer plate was placed (bed height: 4 cm) as described in chapter 5.1. The column was coated with flexible tubing and connected to a cryostat to ensure temperature control. The actual temperature was continuously monitored.

Detritylation, coupling, oxidation or thiolation and capping were repeated for each nucleotide until the desired length (100mer) was achieved as described in chapter 10.1.

The phosphorous protecting groups were removed by treating the solid support on column with a solution of an alkyl amine in acetonitrile for 10 - 60 minutes.

### 10.2.2 Cleavage & Deprotection, Desalting

Cleavage, deprotection and desalting were performed as described in 5.1. The purity and identity were determined via IP-HPLC and LC-MS analysis (see Figure 21 for comparison of IP-RP HPLC analyses of temperature test 10.1-10.3, see Figure 22 B for individual analysis).

### 10.3 Synthesis at >28°C on steel-column:

### 10.3.1 Synthesis

8.35 g of rU CPG were loaded into a 2.7 x 5 cm steel column. On top of the solid support a porous spacer plate was placed (bed height: 4 cm) as described in chapter 5.1. The column was coated with flexible tubing and connected to a cryostat to ensure temperature control. The actual temperature was continuously monitored.

Detritylation, coupling, oxidation or thiolation and capping were repeated for each nucleotide until the desired length (100mer) was achieved as described in chapter 10.1.

The phosphorous protecting groups were removed by treating the solid support on column with a solution of an alkyl amine in acetonitrile for 10 - 60 minutes.

### 10.3.2 Cleavage & Deprotection, Desalting

Cleavage, deprotection and desalting were performed as described in 5.1. The purity and identity were determined via IP-HPLC and LC-MS analysis (see Figure 21 for comparison of IP-RP HPLC analyses of temperature test 10.1-10.3, see Figure 22 C for individual analysis).

Table 14 shows an overview of the results.

**Table 14: Overview of yield and purity data of synthesis 10.1 - 10.3**

| **Synthesis conditions** | **FLP Purity [%, UV]** | **FLP purity increase compared to synthesis at ambient temperature [%, UV]** | **Early-eluting impurities [%, UV]** | **Late-eluting impurities [%, UV]** | **Yield [OD/µmol]** |
|---|---|---|---|---|---|
| Steel column at <18°C °C | 50.28 | 1.08 | 38.75 | 10.96 | 228 |
| Steel column at 18-24 °C | 49.20 | - | 37.25 | 13.56 | 243 |
| Steel column at >28 °C | 32.86 | -16.34 | 44.46 | 22.67 | 201 |

## Claims

1. A method of solid-phase phosphoramidite-synthesis of an oligonucleotide, molecule comprising between 50 and 500 nucleotides, the method comprising at least the following steps:
a) providing a blueprint for the desired oligonucleotide sequence;
b) providing a solid support, preferably a base-loaded controlled pore glass support (CPG) preferably having a pore size from 1400 to 3000 A, functionalized with a linker moiety and providing a plurality of ribonucleotide monomer subunits as well as modified ribonucleotide monomer subunits, preferably 2'-modified and 4' modified ribonucleotide monomer subunits, locked nucleic acids and non-nucleosidic monomers, wherein each of the monomer subunits comprises a protected hydroxyl or specifically protected 5'- hydroxyl group;
c) treating the solid support for a predetermined time with an acidic deblocking solution to expose free 5'-hydroxyl groups;
d) washing the solid support with an appropriate solvent, preferably acetonitrile, to remove the deblocking solution; and the cleaved protecting group.
e) treating the solid support with a coupling mixture comprising an activating reagent and ribonucleotide monomer subunits, each comprising a phosphoramidite group and a blocked hydroxyl group to obtain phosphite triester linked ribonucleotide monomer subunits;
f) washing the solid support with an appropriate solvent, preferably acetonitrile, to remove the coupling mixture;
g) oxidizing or sulfurizing or thiolating the phosphite triester linked ribonucleotide monomer subunits to provide phosphate triester or thiophosphate triester linked ribonucleotide monomer subunits, whereby the oxidation or thiolation is carried out with an appropriate reagent;
h) washing the solid support with an appropriate solvent, preferably acetonitrile, to remove the oxidizing or thiolating solutions;
i) treating the phosphate triester or thiophosphate triester linked ribonucleotide monomer subunits with a mixture of one or more capping reagents to block any uncoupled support-bound 5' hydroxy byproduct, whereby the mixture of one or more capping reagents has less than 15, preferably less than 8, more preferably less than 5 equivalents of capping reagents based on the loading of the solid phase;
j) washing the solid support with an appropriate solvent, preferably acetonitrile, to remove the capping solutions;
k) repeating steps c) to j) until the desired amount of ribonucleotide units is coupled to the solid support to obtain the desired oligonucleotide molecule;
l) treating the solid support with a solution of an alkyl amine, a cyclic or a bicyclic amine in an appropriate solvent, preferably acetonitrile, to remove phosphorous protecting groups;
m) washing the solid support with an appropriate solvent, preferably acetonitrile, to remove the phosphorous deprotection reagent;
n) deprotecting and cleaving the oligoribonucleotide compound from the solid support to obtain the crude oligonucleotide molecule, and
o) optionally, purifying the crude oligonucleotide molecule.

2. The method of claim 1, wherein for up to fifty cycles of the method the coupling step e) is carried out twice before step g) is conducted.

3. The method of claim 1 or 2, wherein the deblocking solution comprises dichloroacetic acid in an amount of between 1 and 10% wt.-%, based on the total weight of the deblocking solution, and the solid support is treated with the deblocking solution for a predetermined time of from 1 to 8 minutes, preferably less than 5 minutes.

4. The method of any one of the preceding claims, wherein the activating reagent of step e) is an acidic activator with a pKₐ < 7.0, and/or the concentration of nucleotide phosphoramidite is between 0.05 to 0.25 M.

5. The method of claim 4, wherein the acidic activator is selected from the group of imidazoles.

6. The method of any one of claims 1 to 5, wherein the capping reagents are selected from N-Methylimidazole/Lutidine or Picoline/Acetonitrile, tert.-Butylphenoxyacetic acid anhydride in acetonitrile, phenoxyacetic acid anhydride in acetonitrile, isobutyric anhydride in acetonitrile and acetic anhydride in acetonitrile.

7. The method of any of the preceding claims, wherein the deprotecting and cleaving step n) is carried out with a solution of ammonia or a basic alkylamine or a mixture of ammonia and a basic alkylamine for 1 to 8 hours at a temperature ≤ 30°C

8. The method of any one of the preceding claims, wherein on the top of the solid support a porous spacer plate is placed, and wherein optionally native controlled pore glass is filled on top of the porous spacer plate.

9. The method of any one of the preceding claims, wherein the thiolation is conducted with (Xanthan hydride) or phenylacetyl disulfide and/or the oxidation is carried out with iodine in pyridine-water.

10. The method of any one of the preceding claims, wherein the synthesis is conducted at a temperature between 10 and 25°C, preferably 14-28°C, more preferably between 15-20°C, most preferably between 18 and 24°C.

11. A method for purifying an oligonucleotide molecule, preferably obtained by a method according to any one of claims 1 to 10, comprising between 50 and 500 nucleotides, synthesized by solid-phase phosphoramidite-synthesis comprising at least the steps
a) providing a crude oligoribonucleotide solution obtained by solid-phase phosphoramidite-synthesis;
b) optionally desalting the crude oligoribonucleotide solution;
c) purifying the desalted oligoribonucleotide solution by a chromatographic method or a combination of chromatographic methods, whereby a buffer having a pH of 3 to 8.5 is used and the chromatographic method is selected from anion exchange chromatography, ion exchange purification, hydrophobic interaction chromatography and/or ion pair - reversed phase purification or a combination thereof;
d) obtaining purified oligonucleotide molecule.
whereby the purification is carried out at a temperature between room temperature and 80°C.

12. The method of claim 11, wherein the phosphoramidite-synthesis is a method according to claims 1 to 10.

13. The method of any one of claims 11 or 12, wherein the buffer is a phosphate buffer or an acetate buffer containing 5 to 30 %, preferably 10-20% of an organic solvent.

14. The method of any one of claims 11 to 13, wherein the purification of step c) is carried out by anion exchange chromatography and is conducted at a temperature of between 45 °C and 80°C, preferably between 50 and 80°C.

15. The method of any one of claims 1 to 14, wherein oligonucleotide molecule is a RNA molecule, preferably a single guide RNA (sgRNA), a prime editing guide RNA (pegRNA), a engineered prime editing guide RNA (epegRNA), a multivalent siRNA passenger strand, a tRNA, an aptamer or a short mRNA.
